# EUROPEAN PATENT APPLICATION

(11) **EP 4 792 809 A1**
(43) Date of publication of application: **19.08.2026**
(21) Application number: 24887942.1
(22) Date of filing: 05.11.2024
(51) Int. Cl.: A61K 31/203, A61K 31/195, C07C 403/20, C07C 215/12, A61P 17/00

(54) **COMPOUND COMPOSITION COMPRISING TRANEXAMIC ACID AND TRETINOIN**

(30) Priority: 06.11.2023 CN 202311464036
(71) Applicant: NANJING INDETEK LABORATORY CO., LTD., Nanjing, Jiangsu 210031 (CN)
(72) Inventor: HU, Tao, Nanjing, Jiangsu 211106 (CN); LIU, Zhenyun, Nanjing, Jiangsu 211106 (CN); LU, Xiqiang, Nanjing, Jiangsu 211106 (CN); WU, Yubo, Nanjing, Jiangsu 211106 (CN); YIN, Miaomiao, Nanjing, Jiangsu 211106 (CN); LEI, Xinqiao, Nanjing, Jiangsu 211106 (CN); YAO, Dongke, Nanjing, Jiangsu 211106 (CN); CHEN, Lei, Nanjing, Jiangsu 211106 (CN)
(74) Representative: Herrero & Asociados, S.L.
(86) International application number: PCT/CN2024/129943
(87) International publication number: WO 2025/098330

(57) **Abstract**

Disclosed are a composition and a formulation. The composition comprises tranexamic acid and a tretinoin alcohol amine or tretinoin fatty amine compound. After the composition is prepared into the formulation, the solubility of tretinoin can be improved, the loss of tretinoin in the formulation process is reduced, the content uniformity of the formulation is improved, and the stability of the formulation is improved. The composition can significantly increase the intradermal retention amount of tretinoin and tranexamic acid, and meanwhile, the cross-skin permeation amount of tranexamic acid is not significantly increased. In the treatment of pigmentation and chloasma, the composition and the formulation can significantly improve the curative effect and avoid aggravating toxic and side effects.

## Description

The present application claims the benefit of:
the patent application No. 202311464036.0, filed with the China national intellectual property administration (CNIPA) on November 6, 2023, entitled "Compound composition including tranexamic acid and tretinoin". The contents of such preceding application are hereby incorporated by reference in its entirety into the present application.

### TECHNICAL FIELD

The present disclosure relates to the field of preparations, specifically relating to a compound composition including tranexamic acid and tretinoin.

### BACKGROUND

The skin, the largest human organ, serves as a primary barrier between the body and the external environment, constantly exposed to the complex external environment and attacked by various harmful factors (heat and cold stimuli, ultraviolet radiation, bacteria, viruses, etc.). Therefore, skin-related abnormalities or diseases often have diverse causes, complex pathogenesis, and varied clinical symptoms.

Topical external preparations are one of the main drugs for the treatment of dermatological diseases. To address the complexity of dermatological diseases, multiple drugs are often used in compound, and are developed into compound preparations. Differences in the physicochemical properties of various drugs make the prescription and process design of compound preparations extremely challenging. For example, when two drugs having different solubility characteristics are designed into a compound preparation, key quality attributes such as compatibility and content uniformity easily come across problems, and thus close attention to the corresponding prescription and process parameters is particularly required. Permeation-enhancing techniques are often used in external preparations to improve drug bioavailability; however, differences in the physicochemical properties of various drugs make permeation-enhancing means collide such that the transdermal absorption of multiple drugs cannot be simultaneously promoted.

Tretinoin is useful for the treatment of various skin diseases and can also improve the skin texture, reduce stains and pigmentation. This drug is poorly soluble, and it exhibits a certain lipid solubility but extremely poor water solubility.

Tranexamic acid (TA) can inhibit the generation of melanin and alleviate various stains and pigmentation. It has good water solubility but poor lipid solubility.

### SUMMARY OF THE INVENTION

To improve the above technical problem, the present disclosure provides a composition including tranexamic acid and a tretinoin complex, and a preparation method and use thereof.

The present disclosure provides a composition, the composition including:
(1) tranexamic acid or a pharmaceutically acceptable salt thereof; and
(2) a tretinoin-alcoholamine complex or a tretinoin-fatty amine complex.

According to an embodiment of the present disclosure, the tretinoin-alcoholamine complex is composed of tretinoin and an alcohol amine compound.

According to an embodiment of the present disclosure, the tretinoin-fatty amine complex is composed of tretinoin and a fatty amine compound.

According to an embodiment of the present disclosure, the fatty amine compound is selected from a C1-20 alkylamine compound, preferably a C1-10 alkylamine compound, e.g. a C9 alkylamine compound, a C8 alkylamine compound, a C7 alkylamine compound, a C6 alkylamine compound, a C5 alkylamine compound, a C4 alkylamine compound, a C3 alkylamine compound, a C2 alkylamine compound, and a C1 alkylamine compound; for example, ethylamine, diethylamine, triethylamine, monomethylamine, dimethylamine, trimethylamine, monopropylamine, dipropylamine, tripropylamine, isopropylamine, diisopropylamine, 1,2-dimethylpropylamine, 1,2-diaminopropane, etc.

According to an embodiment of the present disclosure, the tretinoin-fatty amine complex is composed of tretinoin and triethylamine.

According to an embodiment of the present disclosure, in the tretinoin-fatty amine complex, a molar ratio of the fatty amine compound to tretinoin is in a range of (1-100) : 1, for example, 2 : 1, 2.5 : 1, 3 : 1, 3.5 : 1, 4 : 1, 4.5 : 1, 5 : 1, 5.5 : 1, 6 : 1, 6.5 : 1, 7 : 1, (1-10) : 1, more preferably (1-5) : 1.

According to an embodiment of the present disclosure, a mass ratio of component (1) (in terms of tranexamic acid) and component (2) (in terms of tretinoin) is in a range of 0.01-100 : 0.0001-10, preferably 0.05-50 : 0.0005-5, further preferably 0.1-10 : 0.001-1, for example, 2.5-7.5 (e.g., 2.5, 5, 7.5) : 0.01-0.1 (e.g., 0.01, 0.025, 0.05, 0.1).

According to an embodiment of the present disclosure, in (2) the tretinoin-alcoholamine complex, a molar ratio of the alcohol amine compound to tretinoin is in a range of (1-100) : 1, for example, 2 : 1, 2.5 : 1, 3 : 1, 3.5 : 1, 4 : 1, 4.5 : 1, 5 : 1, 5.5 : 1, 6 : 1, 6.5 : 1, 7 : 1, or (1-10) : 1, more preferably (1-5) : 1.

According to an embodiment of the present disclosure, the alcohol amine compound is selected from formula I as follows: 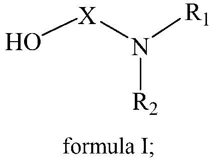
where X is selected from C₁₋₆ alkyl (alkylene), and preferably selected from -CH₂-CH₂-, -CH₂-CH₂-CH₂- and 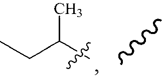 representing a group linkage site; and
R₁ and R₂ are each independently selected from the group consisting of H, C₁₋₆ alkyl, and C₁₋₆ alkyl-OH.

According to an embodiment of the present disclosure, the formula I is selected from diethanolamine, triethanolamine, monoethanolamine (2-hydroxyethylamine), N-methyldiethanolamine, *n*-propanolamine, isopropanolamine, diisopropanolamine, triisopropanolamine, *n*-butanolamine, dibutanolamine, and isobutanolamine.

According to an embodiment of the present disclosure, the complex is in a liquid state at room temperature. In an embodiment, in the complex, the tretinoin may be present partially or entirely in an anionic form, and the alcohol amine compound may be present partially or entirely in a cationic form.

According to an embodiment of the present disclosure, in the complex, part of the tretinoin is present in a molecular form and/or part of the alcohol amine compound is present in a molecular form.

According to an embodiment of the present disclosure, in the complex, tretinoin is present in an [A⁻] form as follows:

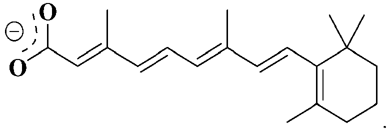

According to an embodiment of the present disclosure, in the complex, the alcohol amine compound is present in a [B⁺] form as shown in formula II: 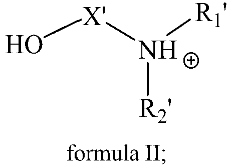
in the formula II, X' is selected from C₁₋₆ alkyl (alkylene), and preferably selected from -CH₂-CH₂-, -CH₂-CH₂-CH₂- and 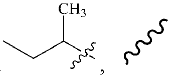 representing a group linkage site; and
R₁' and R₂' are each independently selected from H, C₁₋₆ alkyl, and C₁₋₆ alkyl-OH.

According to an embodiment of the present disclosure, the formula II is selected from a diethanolammonium cation, a triethanolammonium cation, a monoethanolammonium cation (i.e., 2-hydroxyethylammonium cation), a N-methyldiethanolammonium cation, an *n*-propanolammonium cation, an isopropanolammonium cation, a diisopropanolammonium cation, a triisopropanolammonium cation, a *n*-butanolammonium cation, a dibutanolammonium cation, and an isobutanolammonium cation.

According to an embodiment of the present disclosure, the complex is x[B⁺][A⁻], where x may be selected from 1 to 10, e.g., 1, 1.5, 2, 2.5, 3, 3.5, 4, 4.5, 5, 5.5, 6, 6.5, or 7.

According to an embodiment of the present disclosure, examples of a pharmaceutically acceptable salt of tranexamic acid include an alkali metal salt such as a sodium salt and a potassium salt, an alkaline earth metal salt such as a calcium salt and a magnesium salt, a zinc salt, an iron salt, an ammonium salt, a salt formed with a basic amino acid such as arginine, lysine, histidine, and ornithine, and a salt formed with an amine such as monoethanolamine, diethanolamine, and triethanolamine. Among them, a sodium salt, a potassium salt, a triethanolamine salt, and an arginine salt are preferred, and a sodium salt is more preferred.

According to an embodiment of the present disclosure, in the composition, components (1) and (2) are each administered in the form of a pharmaceutical composition.

According to an embodiment of the present disclosure, in the composition, components (1) and (2) are administered simultaneously, sequentially, or at an interval.

According to an embodiment of the present disclosure, a route of administration of the composition includes, but is not limited to, gastrointestinal administration or non-gastrointestinal administration; where the gastrointestinal administration may be oral administration; the non-gastrointestinal administration may be transdermal administration (e.g., topical application administration).

According to an embodiment of the present disclosure, the composition can improve the transdermal property of tretinoin (e.g., increasing the intradermal retention amount without significantly increasing the skin permeation amount).

According to an embodiment of the present disclosure, the composition can improve the transdermal property of component (1) (tranexamic acid) (e.g., increasing the intradermal retention amount without significantly increasing the skin permeation rate).

According to an embodiment of the present disclosure, the composition includes, but is not limited to, a pharmaceutical preparation, a cosmetic, a care product, and a beauty product.

According to an embodiment of the present disclosure, the composition can be administered topically through the skin, for example, in a form selected from a cream, a patch, an ointment, an emulsifiable paste, an emulgel preparation, a gel, and a spray, or administered orally (i.e., as an oral preparation), for example, in a form selected from a tablet, a granule, a capsule, an oral liquid preparation, a pill, a suspension, a dripping pill. Preferably, the composition is an emulsifiable paste, an emulgel preparation, an emulsion preparation, or a gel preparation.

According to an embodiment of the present disclosure, in the composition, a content of component (1) is in a range of 0.01%-20% (in terms of tranexamic acid), preferably 0.1%-15%, further preferably 1%-10%, e.g., 2.5%-7.5%, for example 2.5%, 3.0%, 3.5%, 4.0%, 4.5%, 5.0%, 5.5%, 6.0%, 6.5%, 7.0%, or 7.5%.

According to an embodiment of the present disclosure, in the composition, a content of component (2) is in a range of 0.001%-2% (in terms of tretinoin), preferably 0.01%-1%, further preferably 0.01%-0.5%, e.g., 0.01%, 0.025%, 0.05%, 0.08%, 0.1%, 0.2%, or 0.3%.

According to an embodiment of the present disclosure, the composition is in a dosage strength of 1 g/tube-20 g/tube, e.g., 3 g/tube, 5 g/tube, 10 g/tube, or 15 g/tube.

According to an embodiment of the present disclosure, the composition optionally further includes:
(3) a gel matrix.

According to an embodiment of the present disclosure, the gel matrix is selected from carbomer, such as Carbopol 980 and Carbopol ETD2020.

According to an embodiment of the present disclosure, a content of the gel matrix is in a range of 0.01%-10%, preferably 0.05%-5%, e.g., 0.1%, 0.15%, 0.2%, 0.5%, 1%, 1.5%, 2%, 2.5%, or 3%.

According to an embodiment of the present disclosure, the composition optionally further includes:
(4) a permeation enhancer.

According to an embodiment of the present disclosure, the permeation enhancer is at least one selected from the group consisting of glyceryl monocaprylate, Transcutol P, isopropyl myristate, laurocapram, diethylene glycol monoethyl ether, polyglycerol fatty acid ester, propylene glycol monocaprylate, and caprylocaproyl polyoxylglyceride.

According to an embodiment of the present disclosure, a content of the permeation enhancer is in a range of 0.01%-20%, preferably 0.1%-15%, e.g., 0.5%, 1%, 2%, 3%, 4%, 10%, 12.57%, or 13%.

According to an embodiment of the present disclosure, the composition optionally further includes:
(5) a preservative.

According to an embodiment of the present disclosure, the preservative is at least one selected from the group consisting of benzoic acid, phenoxyethanol, sodium benzoate, methylparaben, ethylparaben, propylparaben, sodium methylparaben, and sodium propylparaben.

According to an embodiment of the present disclosure, a content of the preservative is in a range of 0.001%-2%, preferably 0.005%-1%, e.g., 0.01%, 0.02%, 0.03%, 0.04%, 0.05%, 0.08%, 0.1%, 0.2%, 0.3%, 0.5%, or 0.8%.

According to an embodiment of the present disclosure, the composition optionally further includes:
(6) an antioxidant.

According to an embodiment of the present disclosure, the antioxidant is at least one selected from the group consisting of butylated hydroxyanisole (BHA), dibutyl hydroxytoluene, butylated hydroxyanisole, vitamin C (ascorbic acid), vitamin E, and sodium pyrosulfite.

According to an embodiment of the present disclosure, a content of the antioxidant is in a range of 0.001%-2%, preferably 0.01%-1%, e.g., 0.1%, 0.2%, or 0.3%.

According to an embodiment of the present disclosure, the composition optionally further includes:
(7) a cosolvent.

According to an embodiment of the present disclosure, the cosolvent is selected from an alcohol, the alcohol preferably being at least one selected from the group consisting of glycerol, propylene glycol, 1,2-pentanediol, ethanol, and benzyl alcohol.

According to an embodiment of the present disclosure, a content of the cosolvent includes 0.5%-30%, preferably 1%-20%, e.g., 1%, 2.0%, 2.5%, 5.0%, 10%, 13.5%, 14%, 15%, 16%, 17%, or 18%.

According to an embodiment of the present disclosure, the composition optionally further includes:
(8) an emulsifier.

According to an embodiment of the present disclosure, the emulsifier is at least one selected from the group consisting of Pemulen TR-1 NF, Emulium Delta MB, Tween (e.g., Tween 20, Tween 40, Tween 60, or Tween 80), Span (e.g., Span 20, Span 60, or Span 80), a polyoxyethylene castor oil derivative, poloxamer, Triton, caprylocaproyl polyoxylglyceride, polyethylene glycol stearate, polyoxyethylene-8 beeswax, and lauroyl macrogolglyceride.

In some embodiments, polyethylene glycol stearate is, for example, polyethylene glycol-(32) stearate. In some embodiments, lauroyl macrogolglyceride is, for example, lauroyl macrogolglyceride (6).

In some embodiments, the polyoxyethylene castor oil derivative is selected from polyoxyethylene castor oil or polyoxyethylene hydrogenated castor oil, such as polyoxyethylene 35 castor oil, polyoxyethylene 40 castor oil, polyoxyethylene 54 hydrogenated castor oil, and polyoxyethylene 100 hydrogenated castor oil.

According to an embodiment of the present disclosure, a content of the emulsifier is in a range of 0.1%-30%, preferably 0.2%-20%, e.g., 0.2%, 0.3%, 2%, 5%, 8%, 10%, or 15%.

According to an embodiment of the present disclosure, the composition optionally further includes:
(9) a greasy matrix.

According to an embodiment of the present disclosure, the greasy matrix is at least one selected from the group consisting of a long-chain fatty alcohol, a long-chain fatty acid, a fatty acid ester, and grease. In some embodiments, the long-chain fatty alcohol is selected from cetyl alcohol and octadecanol. In some embodiments, the long-chain fatty acid is selected from stearic acid. In some embodiments, the fatty acid ester is at least one selected from the group consisting of isopropyl myristate, a medium-chain triglyceride, glyceryl monooleate, glyceryl monolinoleate, propylene glycol dicaprylate/dicaprate, polyoxyethylene oleate glyceride, myristyl myristate, isopropyl palmitate, isopropyl linoleate, dodecanol benzoate, isostearyl isostearate, fatty acid lactate, decyl oleate, and octyl palmitate. In some embodiments, the grease is selected from a jojoba oil, a soybean oil, and a linseed oil, further preferably a jojoba oil.

According to an embodiment of the present disclosure, a content of the greasy matrix is in a range of 0.5%-20%, preferably 1.0%-15%, e.g., 1.5%, 2.0%, 5%, 6%, 8%, 10%, or 12%.

According to an embodiment of the present disclosure, the composition optionally further includes:
(10) a rheology modifier.

According to an embodiment of the present disclosure, the rheology modifier is selected from xanthan gum.

According to an embodiment of the present disclosure, a content of the rheology modifier is in a range of 0.01%-0.5%, preferably 0.1%-0.3%, e.g., 0.15%.

According to an embodiment of the present disclosure, the composition optionally further includes at least one selected from the group consisting of a metal ion complexing agent, a co-emulsifier, and a texture modifier.

According to an embodiment of the present disclosure, the co-emulsifier is selected from propylene glycol monocaprylate.

According to an embodiment of the present disclosure, a content of the co-emulsifier is in a range of 0.1%-5%, preferably 1%-3%, e.g., 2.0%.

According to an embodiment of the present disclosure, the texture modifier is selected from cyclopentasiloxane.

According to an embodiment of the present disclosure, a content of the texture modifier is in a range of 1%-5%, e.g., 3.0%.

According to an embodiment of the present disclosure, the metal ion complexing agent is at least one selected from the group consisting of ethylenediaminetetraacetic acid (EDTA), disodium ethylenediaminetetraacetate (EDTA-2Na), tetrasodium ethylenediaminetetraacetate (EDTA-4Na), disodium nitrilotriacetate, sodium tripolyphosphate, sodium hexametaphosphate, and tetrapotassium pyrophosphate, preferably EDTA or EDTA-2Na.

According to an embodiment of the present disclosure, the composition optionally further includes other physiologically acceptable carriers (e.g., a biocompatible material), such as a surfactant, an excipient, a humectant, an emulsification promoter, a suspending agent, a salt or buffer for regulating osmotic pressure, a colorant, a fragrance, a stabilizer, a bactericide, or other conventional supplements.

According to an embodiment of the present disclosure, the composition includes the following components:
2.5%-7.5% (e.g., 2.5%, 5%, or 7.5%) of tranexamic acid;
0.01%-1% (e.g., 0.01%, 0.025%, 0.05%, or 0.1%) of the tretinoin-alcoholamine complex or the tretinoin-fatty amine complex (e.g., tretinoin diethanolamine, tretinoin triethanolamine, or tretinoin triethylamine), in terms of tretinoin;
0.05%-5% (e.g., 0.1%, 0.15%, 0.2%, 0.5%, 1%, 1.5%, 2%, 2.5%, or 3%) of the gel matrix (e.g., Carbopol 980 and/or Carbopol ETD2020);
optionally, 0.1%-15% (e.g., 0.5%, 1%, 2%, 3%, 4%, 10%, 12.57%, or 13%) of the permeation enhancer (e.g., one, two, or more of glyceryl monocaprylate, Transcutol P, isopropyl myristate, laurocapram, diethylene glycol monoethyl ether, polyglycerol fatty acid ester, propylene glycol monocaprylate, and caprylocaproyl polyoxylglyceride);
optionally, 0.005%-1% (e.g., 0.01%, 0.02%, 0.03%, 0.04%, 0.05%, 0.08%, 0.1%, 0.2%, 0.3%, 0.5%, or 0.8%) of the preservative (e.g., one, two, or more of benzoic acid, phenoxyethanol, sodium benzoate, methylparaben, ethylparaben, propylparaben, sodium methylparaben, and sodium propylparaben);
optionally, 0.01%-1% (e.g., 0.1%, 0.2%, or 0.3%) of the antioxidant (e.g., one, two, or more of butylated hydroxyanisole, dibutyl hydroxytoluene, butylated hydroxyanisole, vitamin C, vitamin E, and sodium pyrosulfite);
optionally, 1%-20% (e.g., 1%, 2.0%, 2.5%, 5.0%, 10%, 13.5%, 14%, 15%, 16%, 17%, or 18%) of the cosolvent (e.g., one, two, or more of glycerol, propylene glycol, 1,2-pentanediol, ethanol, and benzyl alcohol);
optionally, 0.2%-20% (e.g., 0.2%, 0.3%, 2%, 5%, 8%, 10%, or 15%) of the emulsifier (e.g., one, two, or more of Pemulen TR-1 NF, Emulium Delta MB, Tween, Span, a polyoxyethylene castor oil derivative, poloxamer, Triton, caprylocaproyl polyoxylglyceride, polyethylene glycol stearate, polyoxyethylene-8 beeswax, and lauroyl macrogolglyceride);
optionally, 1.0%-15% (e.g., 1.5%, 2.0%, 5%, 6%, 8%, 10%, or 12%) of the greasy matrix (e.g., one, two, or more of octadecanol, cetyl alcohol, stearic acid, and a fatty acid ester);
optionally, 0.1%-0.3% (e.g., 0.15%) of the rheology modifier (e.g., xanthan gum);
optionally, 1%-3% (e.g., 2.0%) of the co-emulsifier (e.g., propylene glycol monocaprylate);
optionally, 1%-5% (e.g., 3.0%) of the texture modifier (e.g., cyclopentasiloxane); and
a balance being water.

According to an embodiment of the present disclosure, the composition includes the following components:

| | |
|---|---|
| tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD 2020: | 1% or 0.2% or 0.15%; |
| the antioxidant (e.g., sodium pyrosulfite): | 0.1% or 0.2%; |
| the preservative (e.g., sodium methylparaben, or sodium propylparaben): | 0.02% or 0.04%; |
| the permeation enhancer (e.g., glyceryl monocaprylate): | 2%; |
| the emulsifier (e.g., polyoxyethylene 54 hydrogenated castor oil): | 10%; |
| the cosolvent (e.g., glycerol): | 10%; |
| a balance being water; | |

where the tretinoin-alcoholamine complex or the tretinoin-fatty amine complex for example, tretinoin-diethanolamine complex, tretinoin-triethanolamine complex, and tretinoin-triethylamine complex.

According to an embodiment of the present disclosure, the composition is an emulsifiable paste.

According to an embodiment of the present disclosure, the composition includes the following components:

| | |
|---|---|
| tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD2020: | 1% or 0.2% or 0.15%; |
| the emulsifier (e.g., Pemulen TR-1 NF, or Tween): | 0.3% or 0.6%; |
| the greasy matrix (e.g., octadecanol): | 5%; |
| the antioxidant (e.g., dibutyl hydroxytoluene): | 0.1% or 0.2%; |
| the preservative (e.g., benzyl alcohol): | 1%; |
| the permeation enhancer (e.g., isopropyl myristate): | 12.57%; |
| the cosolvent (e.g., glycerol, or ethanol): | 10% or 12.5%; |
| a balance being water; | |

where the tretinoin-alcoholamine complex or the tretinoin-fatty amine complex, for example, tretinoin-diethanolamine complex, tretinoin-triethanolamine complex, and tretinoin-triethylamine complex.

According to an embodiment of the present disclosure, the composition is an emulgel preparation.

According to an embodiment of the present disclosure, the composition includes the following components:

| | |
|---|---|
| tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD 2020: | 1% or 0.2% or 0.15%; |
| the preservative: | 0.02% or 0.04%; |
| the antioxidant: | 0.1% or 0.2%; |
| the permeation enhancer: | 2%; |
| the cosolvent (e.g., propylene glycol, glycerol, or 1,2-pentanediol): | 15%; |
| a balance being water; | |

where the tretinoin-alcoholamine complex or the tretinoin-fatty amine complex, for example, tretinoin-diethanolamine complex, tretinoin-triethanolamine complex, and tretinoin-triethylamine complex;
the permeation enhancer, for example, is at least one selected from laurocapram, glyceryl monocaprylate, Trancutol P, and isopropyl myristate;
the antioxidant, for example, is at least one selected from dibutyl hydroxytoluene, butylated hydroxyanisole, sodium pyrosulfite, and ascorbic acid;
the preservative, for example, is at least one selected from sodium methylparaben, sodium propylparaben, methylparaben, propylparaben, dibutyl hydroxytoluene, and phenoxyethanol.

According to an embodiment of the present disclosure, the composition is a gel.

According to an embodiment of the present disclosure, the composition includes the following components:

| | |
|---|---|
| tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD2020: | 1% or 0.2% or 0.15%; |
| the permeation enhancer (e.g., Transcutol P): | 5.0%; |
| the emulsifier (e.g., Emulium Delta MB): | 2.0%; |
| the greasy matrix (e.g., a medium-chain triglyceride, or a jojoba oil): | 5% or 1.5% or 6.5%; |
| the co-emulsifier (e.g., propylene glycol monocaprylate): | 2.0%; |
| the texture modifier (e.g., cyclopentasiloxane): | 3.0%; |
| the preservative (e.g., phenoxyethanol): | 0.5%; |
| the cosolvent (e.g., glycerol): | 2%; |
| the rheology modifier (e.g., xanthan gum): | 0.15%; |
| a balance being water; | |

where the tretinoin-alcoholamine complex or the tretinoin-fatty amine complex, for example, tretinoin-diethanolamine complex, tretinoin-triethanolamine complex, and tretinoin-triethylamine complex.

According to an embodiment of the present disclosure, the composition is an emulsion.

The present disclosure further provides use of the above composition in preparation of a drug for preventing and/or treating a skin disease.

According to an embodiment of the present disclosure, the skin disease is selected from the group consisting of chloasma and pigmentation.

The present disclosure further provides a method of preventing and/or treating a skin disease, including: administering an effective amount of the above composition to a patient suffering from a skin disease.

According to an embodiment of the present disclosure, the skin disease is selected from the group consisting of chloasma and pigmentation.

The present disclosure further provides a method for preparing the above composition, the method including steps of:
1) preparing an aqueous phase including tranexamic acid;
2) preparing an oil phase including the tretinoin-alcoholamine complex or the tretinoin-fatty amine complex; and
3) mixing by: adding the aqueous phase into the oil phase, and subjecting a resulting mixture to emulsification and homogenization to obtain the composition.

According to an embodiment of the present disclosure, the aqueous phase further includes a gel matrix.

According to an embodiment of the present disclosure, the aqueous phase optionally further includes one or more of a permeation enhancer, a preservative, an antioxidant, a cosolvent, an emulsifier, a greasy matrix, a rheology modifier, a co-emulsifier, and a texture modifier.

According to an embodiment of the present disclosure, the oil phase optionally further includes one or more of a permeation enhancer, a preservative, an antioxidant, a cosolvent, an emulsifier, a greasy matrix, a rheology modifier, a co-emulsifier, and a texture modifier.

The present disclosure further provides a method for preparing the above composition, the method including steps of:
1) preparing an aqueous phase by: adding tranexamic acid, a preservative, and a gel matrix to a certain amount of water, and stirring a resulting mixture to obtain an aqueous phase;
2) preparing an oil phase by: mixing the tretinoin-alcoholamine complex or the tretinoin-fatty amine complex, a permeation enhancer, an antioxidant, and a cosolvent, and stirring a resulting mixture uniformly to obtain an oil phase; and
3) Mixing by: adding the aqueous phase into the oil phase, and subjecting an obtained mixture to emulsification and homogenization to obtain the composition.

According to an embodiment of the present disclosure, the method specifically includes steps of:
1) dissolving a preservative in a certain amount of water, adding a gel matrix thereto, stirring a resulting mixture at a high rate to disperse uniformly, and then stirring at a low rate to hydrate the resulting mixture;
2) adding an aqueous solution of tranexamic acid thereto, stirring continuously and mixing uniformly so that the gel matrix is gelled, to obtain an aqueous phase;
3) mixing the tretinoin-alcoholamine complex or the tretinoin-fatty amine complex, a permeation enhancer, an antioxidant, and a cosolvent, and stirring a resulting mixture uniformly to obtain an oil phase; and
4) adding the aqueous phase in step 2) into the oil phase in step 3), and subjecting an obtained mixture to emulsification and homogenization to obtain the composition; preferably, the composition is a gel.

According to an embodiment of the present disclosure, the method specifically includes steps of:
1) dissolving a gel matrix in water, stirring, and degassing to prepare a uniform, bubble-free gel matrix phase;
2) adding a preservative to a certain amount of water, adding tranexamic acid thereto after dissolution, and dispersing a resulting mixture uniformly;
3) adding a tranexamic acid solution obtained in step 2) to the gel matrix phase in step 1) to obtain a gel;
4) mixing the tretinoin-alcoholamine complex or the tretinoin-fatty amine complex, an antioxidant, a permeation enhancer, and a certain amount of a cosolvent uniformly to obtain an oil phase; and
5) adding the gel in step 3) into the oil phase in step 4), and subjecting an obtained mixture to emulsification and homogenization to obtain the composition.

According to an embodiment of the present disclosure, the method further includes steps of washing container(s) with water or a cosolvent and mixing a resulting washing liquid.

### Beneficial effects

The present disclosure provides a composition, the composition including tranexamic acid and a tretinoin-alcoholamine complex. The composition can improve the solubility of tretinoin, reduce the loss of tretinoin in the preparation process, improve the content uniformity of a preparation, and improve the stability of the preparation. Moreover, the composition can significantly increase the intradermal retention amount of tretinoin and tranexamic acid, while not significantly increasing the transdermal permeation amount of tranexamic acid. Furthermore, the composition of the present disclosure has lower irritancy, lower toxic side effects, and lower sensitization.

In the treatment of pigmentation and chloasma, the composition provided in the present disclosure can significantly improve the therapeutic effect and avoid aggravating toxic side effects.

### BRIEF DESCRIPTION OF DRAWINGS

FIG. 1 shows comparisons of skin tones on backs of guinea pigs before and after treatment with a compound preparation of a tranexamic acid/tretinoin adduct.
FIG. 2 shows comparisons of skin sections between administration regions and non-administration regions on the back of guinea pigs treated with the compound preparation of the tranexamic acid/tretinoin adduct.
FIG. 3 shows the hydrogen nuclear magnetic resonance (¹H NMR) spectrum of 8[TEA][RA].
FIG. 4 shows the ¹H NMR spectrum of 2[Et₃N][RA].
FIG. 5 shows the prepared chloasma model.
FIG. 6 shows the therapeutic effects of the compound preparations on chloasma.
FIG. 7 shows typical sections when treating chloasma with compound preparations.

### EMBODIMENTS

The technical solutions of the present disclosure will be further explained in detail below in conjunction with specific examples. It should be understood that the following examples are only for illustrating and explaining the present disclosure, and should not be construed as limiting the protection scope of the present disclosure. All technologies implemented based on the above contents of the present disclosure are covered within the intended protection scope of the present disclosure.

Unless otherwise specified, the raw materials and reagents used in the following examples are commercially available products or can be prepared by known methods.

Tranexamic acid was purchased from Yantai Valiant Pharmaceutical Co., Ltd., China. Carbopol 980 (Carbomer 980), Carbopol ETD2020 (Carbomer ETD2020), and Pemulen TR-1 NF were purchased from Lubrizol. Sodium pyrosulfite was purchased from Hunan Erkang Pharmaceutical Co., Ltd., China. Sodium methylparaben, sodium propylparaben, dibutyl hydroxytoluene, and BHA (butylated hydroxyanisole) were purchased from Jiangxi Alpha High-Tech Pharmaceutical Co., Ltd., China. Tretinoin (retinoic acid) and isopropyl myristate were purchased from Shanghai Macklin Biochemical Technology Co., Ltd., China. Glyceryl monocaprylate was purchased from IMCD (China) Co., Ltd. Polyoxyethylene 54 hydrogenated castor oil was purchased from Nanjing Well Pharmaceutical Technology Co., Ltd., China. Glycerol, laurocapram, propylene glycol, and 1,2-pentanediol were purchased from Shandong Ruisheng Pharmaceutical Excipients Co., Ltd., China. Tween 80 was purchased from Sigma. Ethanol, octadecanol (stearyl alcohol), benzyl alcohol, diethanolamine (DEA) (AR), and phenoxyethanol were purchased from Sinopharm Chemical Reagent Co., Ltd., China. Xanthan gum was purchased from Hubei Gedian Humanwell Pharmaceutical Excipients Co., Ltd., China. Transcutol P, Emulium Delta MB, medium-chain triglycerides, and propylene glycol monocaprylate were purchased from Gattefossé. Jojoba oil was purchased from Adamas. Cyclopentasiloxane was purchased from Shanghai Fangdeng Chemical Co., Ltd., China. The commercially available tretinoin ointment (0.05%, 20 g) was purchased from Guangdong Xiangshantang Pharmaceutical Co., Ltd., China. The commercially available hydroquinone emulsifiable paste (2%, 20 g) was purchased from Guangdong Renrenkang Pharmaceutical Co., Ltd., China.

Carbopol 980 and Carbopol ETD2020: carbomer 980 and carbomer ETD2020.

Transcutol P: Diethylene glycol monoethyl ether.

Pemulen TR-1 NF: Carbomer copolymer type B, a high-molecular-weight copolymer of acrylic acid and C10-C30 alkyl acrylate, crosslinked with pentaerythritol allyl ether.

Emulium Delta MB: Cetyl alcohol (and) glyceryl stearate (and) PEG-75 stearate (and) cetyl alcohol polyether-20 (and) stearyl alcohol polyether-20.

### EXAMPLE 1 Preparation of Ordinary Tranexamic Acid/Tretinoin Compound Preparations

### Preparation 1: Ordinary Compound Ointment of Tranexamic Acid/Tretinoin

### Prescription:

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium pyrosulfite | Hunan Erkang | 0.2% |
| | Sodium methylparaben | Jiangxi Alpha | 0.02% |
| | Sodium propylparaben | Jiangxi Alpha | 0.02% |
| | Water | / | 71.66% |
| Oil phase | Tretinoin | Macklin | 0.1% |
| | Glyceryl monocaprylate | IMCD | 2% |
| | Polyoxyethylene 54 hydrogenated castor oil | Nanjing Well | 10% |
| | Glycerol | Shandong Ruisheng | 10% |
| Total | | | 100% |

**Process**: Polyoxyethylene 54 hydrogenated castor oil was heated at 60 °C to melt, and then cooled to 40 °C. The rest of the oil phase raw materials and adjuvants were added thereto for mixing and dissolution. Tranexamic acid was dissolved in about 40% of water. The rest of the aqueous phase adjuvants were mixed with the remaining water and then Carbopol 980 was added thereto to disperse uniformly. An aqueous solution of tranexamic acid was mixed with the aqueous phase adjuvants, and a resulting mixture was mechanically stirred for 30 min to obtain an aqueous phase. The two phases were mixed and homogenized at 1000 rpm for 2 min using a homogenizer. After being mechanically stirred (four-blade or anchor type) for 30 min until being uniformly mixed, the resulting mixture was filled into aluminum-plastic composite tubes to obtain the ointment.

### 1.2. Preparation 2: Ordinary Compound emulgel of Tranexamic acid/Tretinoin

### Prescription:

| **Ingredient/Manufacturer** | | | **Proportion** |
|---|---|---|---|
| Aqueous phase | Tranexamic acid | Yantai Valiant | 5% |
| | Carbopol ETD 2020 | Lubrizol | 0.2% |
| | Pemulen TR-1 NF | Lubrizol | 0.3% |
| | Tween 80 | Sigma | 0.3% |
| | Water | / | 62.93% |
| Oil phase | Tretinoin | Macklin | 0.1% |
| | Ethanol | Sinopharm | 2.5% |
| | Octadecanol (stearyl alcohol) | Sinopharm | 5% |
| | Glycerol | Shandong Ruisheng | 10% |
| | Dibutyl hydroxytoluene | Jiangxi Alpha | 0.1% |
| | Benzyl alcohol | Sinopharm | 1% |
| | Isopropyl myristate | Macklin | 12.57% |
| Total | | | 100% |

**Process**: Each adjuvant was weighed out according to the prescribed amount; tranexamic acid was dissolved in about 40% of water; after the rest of the aqueous phase materials were mixed uniformly, the tranexamic acid solution was added thereto, and a resulting mixture was mechanically stirred for 30 min until being mixed uniformly, and carbomer ETD2020 was gelled; the resulting aqueous phase was placed in a 40 °C water bath for later use. Tretinoin and ethanol were weighed out according to the prescribed amounts and mixed. Isopropyl myristate and benzyl alcohol were weighed out and added thereto, followed by mixing uniformly. Octadecanol and dibutyl hydroxytoluene were weighed out and added to the oil phase, and then the resulting mixture was heated in a 60 °C water bath to dissolve, followed by cooling to 40 °C. While being kept in a 40 °C water bath, the oil phase was added to the aqueous phase, and a resulting mixture was homogenized with a high-shear homogenizer for 3 min, and mixed by mechanical stirring (four-blade or anchor type) to form a semi-solid emulgel. After cooling, the emulgel was filled into aluminum-plastic composite tubes.

### 1.3. Preparation 3: Ordinary Compound Gel of Tranexamic acid/Tretinoin

### Prescription:

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.66% |
| Oil phase | Tretinoin | Macklin | 0.1% |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

**Process**: Each adjuvant was weighed out according to the prescribed amount; tranexamic acid was dissolved in about 40% of water; after the preservatives (sodium methylparaben and sodium propylparaben) were dissolved in the remaining water, Carbopol 980 was added thereto, and a resulting mixture was stirred at a high rate of 200 rpm for 5 min to disperse uniformly; the resulting mixture was stirred at a low rate of 50 rpm for 50 min to hydrate the carbomer; the tranexamic acid solution was added thereto, and a resulting mixture was mechanically stirred for 30 min to mix uniformly and the carbomer was gelled; the oil phase adjuvants were mixed and stirred uniformly to form a homogeneous oil phase. The aqueous phase was added to the oil phase, and the resulting mixture was homogenized with a high-shear homogenizer at 5000 rpm for 3 min. The resulting mixture was mechanically stirred at a low rate for 30 min to form a homogeneous gel. The resulting gel was filled into aluminum-plastic composite tubes.

### 1.4. Preparation 4: Ordinary Compound Emulsion of Tranexamic acid/Tretinoin

### Prescription:

| **Ingredient/Manufacturer** | | | **Proportion** |
|---|---|---|---|
| Aqueous phase | Tranexamic acid | Yantai Valiant | 5% |
| | Carbomer 980 | Lubrizol | 0.15% |
| | Glycerol | Shandong Ruisheng | 2.0% |
| | Xanthan gum | Gedian Humanwell | 0.15% |
| | Transcutol P | Gattefossé | 5.0% |
| | Water | / | 72.6% |
| Oil phase | Tretinoin | Macklin | 0.1% |
| | Emulium Delta MB | Gattefossé | 2.0% |
| | Medium-chain triglyceride | Gattefossé | 6.0% |
| | Propylene glycol monocaprylate | Gattefossé | 2.0% |
| | Jojoba oil | Adamas | 1.5% |
| | Cyclopentasiloxane | Fangdeng Chemical | 3.0% |
| | Phenoxyethanol | Sinopharm | 0.5% |
| Total | | | 100% |

**Process**: All adjuvants were weighed out. Xanthan gum and carbomer 980 were dispersed in glycerol and Transcutol P. After a part of water was added thereto, the resulting mixture was stirred and mixed uniformly in an 80 °C water bath. Tranexamic acid was dissolved in the remaining water and then added to the aqueous phase, and the resulting mixture was mechanically stirred to mix uniformly and carbomer 980 was gelled. All components in the oil phase were mixed and kept at 80 °C. The oil phase was added to the aqueous phase and homogenized at 8000 rpm for 3 min. The resulting mixture was cooled to 40-50 °C, and phenoxyethanol was added thereto. The resulting mixture was mechanically stirred for 30 min to mix uniformly, and dispensed into aluminum tubes.

### EXAMPLE 2 Preparation of Compound Preparations of Tranexamic acid/Tretinoin-diethanolamine Adduct

### 2.1. Preparation of Tretinoin Adduct

**Raw materials and reagents**: Retinoic acid (RA) (AR, Shanghai Aladdin Biochemical Technology Co., Ltd., China), diethanolamine (DEA) (AR, Sinopharm Chemical Reagent Co., Ltd., China), and ethanol (AR, Sinopharm Chemical Reagent Co., Ltd., China).

### Prescription:

| **Material** | **Amount** | **Solvent** | **Solvent amount** |
|---|---|---|---|
| Retinoic acid (RA) | 6.0 g | Ethanol | 30 mL-60 mL |
| Diethanolamine (DEA) | 4.2 g | Ethanol | 5 mL |

The molar ratio of diethanolamine (DEA) to retinoic acid (RA) was 2 : 1.

### Process:

(1) Retinoic acid was weighed out according to the prescribed amount and placed in a round-bottom flask, ethanol was added thereto, and a resulting mixture was mixed by stirring; retinoic acid could not be completely dissolved and was present in a suspension state; and the round-bottom flask was wrapped with aluminum foil to block light.

(2) Diethanolamine was weighed out according to the prescribed amount and placed in a container wrapped with aluminum foil, and ethanol was added thereto followed by stirring to dilute.

The diethanolamine dilution obtained in step (2) was added dropwise to the mixture obtained in step (1) with stirring. The container was washed with about 5 mL of ethanol, and the resulting liquid was also added to the flask, followed by stirring.

After all the additions were completed, the resulting solution should be present in a clear orange-red color. The stirring was continued for 4 h.

The solvent was removed using a rotary evaporator. The obtained sample was stored in a sealed, light-blocked container.

The ¹H NMR, infrared spectrum (IR), and Raman spectra of the retinoic acid-diethanolamine complex (2[DEA][RA]) can be found in patent application CN202210853741.9. The tretinoin adduct in Preparations 5-9 below is a retinoic acid-diethanolamine complex (2[DEA][RA]).

### 2.2 Preparation 5: Compound Ointment of Tranexamic Acid/Tretinoin Adduct

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium pyrosulfite | Hunan Erkang | 0.2% |
| | Sodium methylparaben | Jiangxi Alpha | 0.02% |
| | Sodium propylparaben | Jiangxi Alpha | 0.02% |
| | Water | / | 71.59% |
| Oil phase | Tretinoin adduct | / | 0.17% (0.1% in terms of tretinoin) |
| | Glyceryl monocaprylate | IMCD | 2% |
| | Polyoxyethylene 54 hydrogenated castor oil | Nanjing Well | 10% |
| | Glycerol | Shandong Ruisheng | 10% |
| Total | | | 100% |

**Process**: Polyoxyethylene 54 hydrogenated castor oil was heated at 60 °C to melt, and then cooled to 40 °C. The rest of the oil phase raw materials and adjuvants were added thereto for mixing and dissolution. Tranexamic acid was dissolved in about 40% of water. The rest of the aqueous phase adjuvants were mixed with the remaining water and then Carbopol 980 was added thereto to disperse uniformly. An aqueous solution of tranexamic acid was mixed with the aqueous phase adjuvants, and the resulting mixture was mechanically stirred for 30 min to obtain an aqueous phase. The two phases were mixed and homogenized at 1000 rpm for 2 min using a homogenizer. After being mechanically stirred (four-blade or anchor type) for 30 min until being uniformly mixed, the resulting mixture was filled into aluminum-plastic composite tubes to obtain the ointment.

### 2.3 Preparation 6: Compound Emulgel of Tranexamic Acid/Tretinoin Adduct

### Prescription:

| **Ingredient/Manufacturer** | | | **Proportion** |
|---|---|---|---|
| Aqueous phase | Tranexamic acid | Yantai Valiant | 5% |
| | Carbopol ETD 2020 | Lubrizol | 0.2% |
| | Pemulen TR-1 NF | Lubrizol | 0.3% |
| | Tween 80 | Sigma | 0.3% |
| | Water | / | 62.86% |
| Oil phase | Tretinoin adduct | / | 0.17% (0.1% in terms of tretinoin) |
| | Ethanol | Sinopharm | 2.5% |
| | Octadecanol (stearyl alcohol) | Sinopharm | 5% |
| | Glycerol | Shandong Ruisheng | 10% |
| | Dibutyl hydroxytoluene | Jiangxi Alpha | 0.1% |
| | Benzyl alcohol | Sinopharm | 1% |
| | Isopropyl myristate | Macklin | 12.57% |
| Total | | | 100% |

**Process**: Each adjuvant was weighed out according to the prescribed amount; tranexamic acid was dissolved in about 40% of water; after the rest of the aqueous phase materials were mixed uniformly, the tranexamic acid solution was added thereto, and the resulting mixture was mechanically stirred for 30 min to mix uniformly, and carbomer ETD2020 was gelled; and the resulting aqueous phase was placed in a 40 °C water bath for later use. The tretinoin adduct and ethanol were weighed out according to the prescribed amounts and mixed. Isopropyl myristate and benzyl alcohol were weighed out, and added thereto, followed by mixing uniformly. Octadecanol and dibutyl hydroxytoluene were weighed out and added to the oil phase, and the resulting mixture was then heated in a 60 °C water bath to dissolve, followed by cooling to 40 °C. While being kept in a 40 °C water bath, the oil phase was added to the aqueous phase, and the resulting mixture was homogenized with a high-shear homogenizer for 3 min, and mixed by mechanical stirring (four-blade or anchor type) to form a semi-solid emulgel. After cooling, the resulting emulgel was filled into aluminum-plastic composite tubes.

### 2.4 Preparation 7: Compound Gel of Tranexamic Acid/Tretinoin Adduct

### Prescription:

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

**Process**: Each adjuvant was weighed out according to the prescribed amount; tranexamic acid was dissolved in about 40% of water; after the preservative was dissolved in the remaining water, Carbopol 980 was added thereto, and the resulting mixture was stirred at a high rate of 200 rpm for 5 min to disperse uniformly; the resulting mixture was stirred at a low rate of 50 rpm for 50 min to hydrate the carbomer; the tranexamic acid solution was added thereto, and the resulting mixture was mechanically stirred for 30 min to mix uniformly and the carbomer was gelled; the oil phase adjuvants were added thereto and mixed uniformly with stirring to form a homogeneous oil phase. The aqueous phase was added to the oil phase, and the resulting mixture was homogenized with a high-shear homogenizer at 5000 rpm for 3 min. The resulting mixture was mechanically stirred at a low rate for 30 min to form a homogeneous gel. The gel was filled into aluminum-plastic composite tubes.

### 2.5 Preparation 8: Compound Emulsion of Tranexamic Acid/Tretinoin Adduct

### Prescription:

| **Ingredient/Manufacturer** | | | **Proportion** |
|---|---|---|---|
| Aqueous phase | Tranexamic acid | Yantai Valiant | 5% |
| | Carbomer 980 | Lubrizol | 0.15% |
| | Glycerol | Shandong Ruisheng | 2.0% |
| | Xanthan gum | Gedian Humanwell | 0.15% |
| | Transcutol P | Gattefossé | 5.0% |
| | Water | / | 72.53% |
| Oil phase | Tretinoin adduct | / | 0.17% (0.1% in terms of tretinoin) |
| | Emulium Delta MB | Gattefossé | 2.0% |
| | Medium-chain triglyceride | Gattefossé | 6.0% |
| | Propylene glycol monocaprylate | Gattefossé | 2.0% |
| | Jojoba oil | Adamas | 1.5% |
| | Cyclopentasiloxane | Fangdeng Chemical | 3.0% |
| | Phenoxyethanol | Sinopharm | 0.5% |
| Total | | | 100% |

**Process**: All adjuvants were weighed out. Xanthan gum and carbomer 980 were dispersed in glycerol and Transcutol P. After a part of water was added thereto, the resulting mixture was stirred and mixed uniformly in an 80 °C water bath. Tranexamic acid was dissolved in the remaining water and the resulting solution was then added to the aqueous phase, and the resulting mixture was mechanically stirred to mix uniformly and carbomer 980 was gelled. All components in the oil phase were mixed and kept at 80 °C. The oil phase was added to the aqueous phase and the resulting mixture was homogenized at 8000 rpm for 3 min, followed by cooling to 40-50 °C; and phenoxyethanol was added thereto. The resulting mixture was mechanically stirred for 30 min to mix uniformly, and dispensed into aluminum tubes.

### EXAMPLE 3 Comparison of IVPT (in vitro permeation test) of Preparations 1-8

### 3.1 IVPT method

IVPT was conducted on isolated skin from Bama miniature pigs (age ≤ 3 months) in a Franz diffusion cell. The receiving fluid was collected at different time points (1 h, 2 h, 4 h, 6 h). After the test was completed, the skin was washed and then the tissue was broken up to obtain an intradermal retention extract. The obtained samples were tested by UPLC-MS to obtain the contents of tranexamic acid and tretinoin in the samples.

### 3.2 IVPT Results

**Table 1. IVPT Results of Different Compound Preparations**

| **Dosage form** | **Preparation** | **Tretinoin (ng/cm²)** | | **Tranexamic acid (µg/cm²)** | |
|---|---|---|---|---|---|
| | | **Intradermal retention amount per unit area** | **Permeation amount per unit area** | **Intradermal retention amount per unit area** | **Permeation amount per unit area** |
| Ointment | Preparation 1: Ordinary compound ointment | 40.38±20.33 | ND | 37.98±18.69 | 292.38±95.20 |
| | Preparation 5: Adduct compound ointment | 210.34±94.54 | ND | 76.35±12.61 | 357.59±73.60 |
| Emulgel | Preparation 2: Ordinary compound emulgel | 53.65±37.41 | ND | 29.94±6.80 | 330.43±111.3 2 |
| | Preparation 6: Adduct compound emulgel | 265.33±96.93 | ND | 126.47±25.42 | 365.96±96.16 |
| Gel | Preparation 3: Ordinary compound gel | 43.06±26.78 | ND | 36.43±11.85 | 315.49±100.5 6 |
| | Preparation 7: Adduct compound gel | 376.88±77.03 | ND | 133.01±43.45 | 379.05±58.19 |
| Emulsion | Preparation 4: Ordinary compound emulsion | 37.12±11.44 | ND | 39.80±12.96 | 352.58±63.58 |
| | Preparation 8: Adduct compound emulsion | 257.33±72.63 | ND | 196.63±69.89 | 372.34±85.17 |

| | | | | | |
|---|---|---|---|---|---|
| ND- indicates "Not detected". | | | | | |

The IVPT results in Table 1 show that the adduct compound preparations (Preparations 5-8) had a higher tretinoin permeation amount; various dosage forms, such as an ointment, an emulgel, a gel, and an emulsion, had a tretinoin permeation amount 5.2, 4.9, 8.6, and 6.9 times that of ordinary compound preparations (Preparations 1-4), respectively.

Furthermore, the tretinoin adduct also had an effect in enhancing the permeation of tranexamic acid; the intradermal retention amount of tranexamic acid in Preparations 5-8 was 2.0, 4.2, 3.7, and 4.9 times that of preparations (Preparations 1-4), respectively. Meanwhile, there was no significant difference in the permeation amount of tranexamic acid between the adduct compound preparations and the ordinary compound preparations.

### EXAMPLE 4 Comparison of Content and Content Uniformity of Preparations 1-8

Tretinoin was in a low content in the preparations. The adsorption by the container or stirring paddles during mixing would greatly affected the content. Moreover, a low content also easily resulted in an uneven content.

Three samples were randomly selected from each preparation to determine the content, and to calculate a RSD% of the intra-batch content.

**Table 2 Content and Content Uniformity of Different Compound Preparations**

| **Classification** | **Name** | **Tretinoin content** | | **Tranexamic acid content** | |
|---|---|---|---|---|---|
| | | **Average/%** | **RSD%** | **Average/%** | **RSD%** |
| Ordinary tranexamic acid-tretinoin compound preparation | Preparation 1: Ointment | 89.59 | 8.88 | 99.14 | 4.74 |
| | Preparation 2: Emulgel | 87.59 | 6.26 | 99.64 | 5.20 |
| | Preparation 3: Gel | 89.98 | 5.68 | 97.65 | 3.32 |
| | Preparation 4: Emulsion | 89.19 | 7.13 | 96.23 | 3.18 |
| Tranexamic acid-tretinoin adduct compound preparation | Preparation 5: Ointment | 96.76 | 4.17 | 98.40 | 4.15 |
| | Preparation 6: Emulgel | 95.96 | 2.30 | 97.59 | 3.37 |
| | Preparation 7: Gel | 97.98 | 3.89 | 99.70 | 2.99 |
| | Preparation 8: Emulsion | 96.24 | 3.15 | 98.56 | 2.02 |

From the results of content and content uniformity in Table 2, it can be seen that: in all of the four ordinary compound preparations of tranexamic acid and tretinoin, the content of tretinoin was low, and the content uniformity thereof was poor; however, by first preparing tretinoin into an adduct and then preparing the adduct in compound with tranexamic acid into a compound preparation, the loss of tretinoin caused by transfer and adsorption during the preparation was reduced, so the content of tretinoin in the resulting preparation was significantly increased, and the content uniformity thereof was also significantly improved.

### EXAMPLE 5 Comparison of Drug Distribution in Preparations 1-8

Samples of Preparations 1-8 were observed using a microscope. In the microscopy field of view, in the ordinary compound preparations, part of tretinoin was dispersed in the state of solid particles in the preparations, e.g., needle-like crystals; while in the adduct compound preparations, tretinoin was mainly dispersed in the state of oil droplets in the preparations. As can be seen, the distribution of tretinoin in the adduct compound preparations was more uniform and stable.

### EXAMPLE 6 Scale-up Production of Compound Gel of Tranexamic Acid and Tretinoin-Diethanolamine Complex (Preparation 9)

### 6.1 Prescription for Scale-up Production

| **Ingredient/Manufacturer** | | | **Proportion** | **Amount for obtaining 5 kg product** |
|---|---|---|---|---|
| Aqueous phase | Tranexamic acid | Yantai Valiant | 5% | 250 g |
| | Carbopol 980 | Lubrizol | 1% | 50 g |
| | Sodium methylparaben | Jiangxi Alpha | 0.02% | 1 g |
| | Sodium propylparaben | Jiangxi Alpha | 0.02% | 1 g |
| | Water | / | 76.675% | 3833.75 g |
| Oil phase | Tretinoin adduct | / | 0.085% (0.05% in terms of tretinoin) | 4.25 g |
| | Dibutyl hydroxytoluene | Jiangxi Alpha | 0.2% | 10 g |
| | Laurocapram | Shandong Ruisheng | 2% | 100 g |
| | Propylene glycol | Shandong Ruisheng | 15% | 750 g |
| Total | | | 100% | 5000 g |

### 6.2 Scale-up Production Process:

The raw materials and adjuvants were weighed out according to the prescribed amounts. To a water tank, about 20% of water was added, followed by stirring at 30 rpm. A Carbomer 980 powder was added thereto with stirring and after the addition, the resulting mixture was stirred for 10 min. The stirring rate was further increased to 60 rpm and the stirring was kept for 1 h. A negative pressure of -0.08 MPa was set and the carbomer solution was drawn to a main pot by vacuum negative pressure within about 3 min. The container was washed with about 10% purified water with stirring at a negative pressure of -0.08 MPa. Degassing was performed in the main pot with stirring at 25 rpm for 30 min, i.e., obtaining a homogeneous, bubble-free carbomer phase.

A prescribed amount of laurocapram was added to a beaker, and a tretinoin adduct and dibutyl hydroxytoluene were added to laurocapram with magnetic stirring to dissolve. The resulting solution was added to 10% propylene glycol, and the resulting mixture was stirred until being well mixed.

Sodium methylparaben and sodium propylparaben were added to about 40% of water, followed by stirring at 400 rpm to dissolve. After the dissolution, a prescribed amount of tranexamic acid was added with stirring to ensure complete dispersion.

The tranexamic acid solution was added to the carbomer phase; the container for the tranexamic acid phase was washed with the remaining water, and the resulting liquid was also added to the carbomer phase. An emulsification homogenization tank was set at a negative pressure of -0.08 MPa, and the stirring was conducted at a stirring rate of 50 rpm and an auxiliary stirring rate of 200 rpm for about 1 h until the material in the tank became a clear, transparent gel free of white impurities.

A tretinoin adduct phase solution was added to the aqueous phase mixed material; and the container for the tretinoin adduct phase was washed with 5% propylene glycol, and the resulting liquid was also added to the mixed material. The emulsification homogenization tank was set at a negative pressure of -0.08 MPa, and the stirring was conducted at a stirring rate of 50 rpm and an auxiliary stirring rate of 200 rpm for about 120 min.

### 6.3 Content and Content Uniformity of Scale-up Batches

**Table 3 Content Uniformity of Tretinoin in Scale-up Batches**

| **Sampling location** | **Content (%)** | **Average** | **RSD (%)** |
|---|---|---|---|
| Paddle edge-1 | 104.5 | 104.7 | 0.3 |
| Paddle edge-2 | 104.8 | | |
| In the middle of paddle-1 | 105.2 | 105.4 | 0.3 |
| In the middle of paddle-2 | 105.5 | | |
| In pot-1 | 104.4 | 105.2 | 1.1 |
| In pot-2 | 105.9 | | |
| Pot edge-1 | 103.9 | 104.0 | 0.1 |
| Pot edge-2 | 104.0 | | |

**Table 4 Content Uniformity of Tranexamic Acid of Scale-up Batches**

| **Batch No.** | **Content (%)** | **Average** | **RSD (%)** |
|---|---|---|---|
| Paddle edge-1 | 109.3 | 109.0 | 0.4 |
| Paddle edge-2 | 108.7 | | |
| In the middle of paddle-1 | 108.0 | 108.1 | 0.2 |
| In the middle of paddle-2 | 108.2 | | |
| In pot-1 | 108.3 | 107.9 | 0.6 |
| In pot-2 | 107.4 | | |
| Pot edge-1 | 107.6 | 107.3 | 0.5 |
| Pot edge-2 | 106.9 | | |

**Table 5 Uniformity of Tretinoin and Tranexamic Acid of Scale-up Batches in Container**

| **Batch No.** | **Tretinoin content (%)** | **Tranexamic acid content (%)** |
|---|---|---|
| Upper part of container | 105.8 | 104.8 |
| Middle part of container | 105.9 | 105.2 |
| Lower part of container | 107.0 | 105.8 |
| NMT% (10%) | 1.2 | 1.0 |

Tables 3-5 show that the compound gel of the tranexamic acid and tretinoin complex prepared by the 5 kg scale-up process exhibited uniform content distribution of tretinoin and tranexamic acid within the preparation tank.

### 6.4 Related Substances of Scale-up Batch

**Table 6 Tretinoin-Related Substances of Scale-up Batch**

| **Name** | **Isotretinoin%** | **Maximum unknown single impurity %** | **Total impurities %** |
|---|---|---|---|
| Scale-up batch | 0.113 | 0.052 | 0.659 |

**Table 7 Tranexamic Acid-Related Substances of Scale-up Batch**

| **Name** | **Aminomethylbenzoic acid %** | **Maximum unknown single impurity %** | **Total impurities %** |
|---|---|---|---|
| Scale-up batch | ND | ND | ND |

Tables 6-7 show that the compound gel of the tranexamic acid and tretinoin complex prepared by the 5 kg scale-up process had a total impurity content within a controllable range, and all indicators met the quality standards, with good stability.

### 6.5 Stability Data of Scale-up Batch in Accelerated Test (30 °C ± 2 °C/65% RH ± 5% RH)

**Table 8 Acceleration Test Results of Scale-up Batch**

| **Acceleration conditions** | 30 °C ± 2 °C/65% RH ± 5% RH | | | |
|---|---|---|---|---|
| **Assessment item** | | Time (in unit of month) | | |
| | | 0 | 1 | 3 |
| **Trait** | | This product is a slightly yellow gel | This product is a slightly yellow gel | This product is a slightly yellow gel |
| **Related substance-tretinoin** | Isotretinoin% | 0.117% | 0.486% | 0.544% |
| | Maximum unknown single impurity % | 0.022% | 0.036% | 0.039% |
| | Total impurities % | 0.209% | 0.570% | 0.638% |
| **Related substance-tranexamic acid** | Aminomethylbenzoic acid % | ND | ND | ND |
| | Maximum unknown single impurity % | ND | ND | ND |
| | Total impurities ≤ 1.0% | ND | ND | ND |
| **Content** | Tretinoin % | 108.7% | 106.2% | 105.7% |
| | Tranexamic acid % | 106.3% | 104.8% | 104.1% |

The accelerated test results show that after 3 months of accelerated storage, all indicators of the prepared adduct compound preparations met the quality standards and exhibited good stability. **EXAMPLE 7 Whitening Effect Test of Adduct Compound Preparation 7**

Male calico guinea pigs were selected as model animals, weighing between 300 g and 350 g. The effect of the compound preparation of the tranexamic acid and tretinoin adduct (Preparation 7) on melanin in brown-skinned guinea pigs was investigated.

### 7.1. Test protocol

**Grouping**: Guinea pigs were acclimated for 7 days. Hair on the back was removed using a hair removal cream on day before grouping. The hair removal area was 3*3 cm². Hair removal was adjusted according to hair growth during the test. 40 guinea pigs were randomly divided into 5 groups, each of the groups had 8 guinea pigs. The medication details for each group are shown in Table 9.

**Table 9 Grouping for Efficacy Test of Drugs**

| No. | Drugs | Dosage Strength | Manufacturer |
|---|---|---|---|
| **T1** | Tranexamic acid/tretinoin adduct compound gel | 5%/0.1%, 10 g | / |
| **T2** | Commercially available tretinoin ointment | 0.05%, 20 g | Guangdong Xiangshantang Pharmaceutical, China |
| **T3** | Commercially available hydroquinone emulsifiable paste | 2%, 20 g | Guangdong Renrenkang Pharmaceutical, China |
| **T4** | Blank group | Blank gel | / |

**Administration**: Each guinea pig was applied with a drug evenly on the hair removal area once daily for 4 weeks. A thin, even layer of drug was applied to each group, covering the hair removal area. Any residual drug was washed off 12 h after each administration.

**Symptom observation**: The skin condition of the administration region was observed and recorded before each administration, to determine whether there is any skin irritancy.

**Skin Tone Chart Detection**: After animal grouping, detection was conducted on each animal using a skin tone chart and the skin tone of the administration region was recorded. The skin tone of the administration regions was measured using a skin tone chart weekly and recorded during the administration until the end of administration. The skin tone difference in each group before and after drug administration was compared.

**Skin Colorimetry and Dermoscopy Detection**: Skin colorimetry was detected using a skin colorimeter before administration and four weeks after administration.

**Fotana-Masson Staining of Skin Sections**: Four weeks after administration, skin samples were taken from the administration and non-administration regions on the back of the guinea pigs for paraffin sectioning, and then subjected to Fotana-Masson staining to assess changes in melanin.

### 7.2. Test results

### 7.2.1 Skin Tone Chart Detection

### Table 10 Comparison of Mean Values of Skin Tone Chart Readings of Animals in Each Group before and after Administration

| Group | D0 | D28 | ΔD |
|---|---|---|---|
| **T1: Adduct compound gel** | 23.33 | 19.33 | -4.00 |
| **T2: Commercially available hydroquinone emulsifiable paste** | 11.00 | 10.50 | -0.50 |
| **T3: Commercially available tretinoin emulsifiable paste** | 16.67 | 15.34 | -1.33 |
| **T4: Blank control** | 20.75 | 20.00 | -0.75 |

A lower color chart reading represents a whiter skin. The mean values of skin tone chart readings for each group before and after administration are shown in Table 10: The adduct compound gel had a more significant effect in reducing melanin, better than the commercially available tretinoin emulsifiable paste, and better than the positive control drug hydroquinone.

### 7.2.2 Skin Tone Detection

**Table 11 L*Mean (Mean ± SD) for Each Group of Animals**

| Group | D0 | D28 | ΔL* |
|---|---|---|---|
| **T1: Adduct compound gel** | 26.55±6.69 | 43.43±1.28 | 16.88 |
| **T2: Commercially available hydroquinone emulsifiable paste** | 45.33±13.92 | 52.17±6.73 | 6.84 |
| **T3: Commercially available tretinoin emulsifiable paste** | 44.65±12.05 | 47.07±3.60 | 2.42 |
| **T4: Blank control** | 41.24±8.29 | 44.09±2.51 | 2.85 |

The L* value measured by a colorimeter was used to evaluate the pharmaceutical effect. The L* value represents the light and dark balance of the skin, primarily influenced by melanin; a higher L* value indicates a lighter skin tone. Table 11 shows that the L* values of all animal groups increased after administration, indicating varying degrees of skin lightening. The adduct compound gel exhibited a strong effect in reducing melanin, greater than that of the commercially available hydroquinone positive control group.

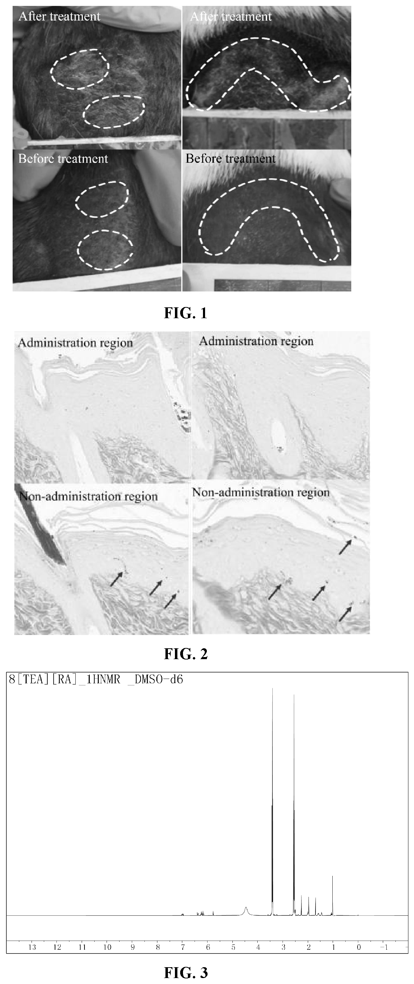

As can be seen from FIG. 1, the skin tones of guinea pigs significantly lightened after treatment with the tranexamic acid/tretinoin adduct compound preparation, compared with those of guinea pigs before treatment.

As can be seen from FIG. 2, the melanin in the epidermal layer significantly decreased after treatment with the tranexamic acid/tretinoin adduct compound preparation, compared with that before treatment.

### Example 8 Validation of Permeation-Enhancing Effect of Adduct

### 8.1 Preparation 10: Adduct-Free Process Compound Gel (Diethanolamine, a ligand, added to the prescription as an adjuvant)

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin | Macklin | 0.1% |
| | Diethanolamine | Sinopharm | 0.07% |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

**Process**: Each adjuvant was weighed out according to the prescribed amount; tranexamic acid was dissolved in about 40% of water; after the preservatives (sodium methylparaben and sodium propylparaben) were dissolved in the remaining water, Carbomer 980 was added thereto; and the resulting mixture was stirred at a high rate of 200 rpm for 5 min to disperse uniformly, and then stirred at a low rate of 50 rpm for 50 min to hydrate the carbomer. The tranexamic acid solution was added thereto, and the resulting mixture was mechanically stirred for 30 min to mix uniformly and the carbomer was gelled. The oil phase adjuvants were added thereto and the resulting mixture was mixed uniformly by stirring to form a homogeneous oil phase. The aqueous phase was added to the oil phase, and the resulting mixture was homogenized with a high-shear homogenizer at 5000 rpm for 3 min. The resulting mixture was mechanically stirred at a low rate for 30 min to form a homogeneous gel. The gel was filled into aluminum-plastic composite tubes.

### 8.2. IVPT

### 8.2.1 IVPT method

IVPT was conducted on isolated skin from Bama miniature pigs (age ≤ 3 months) in a Franz diffusion cell. The receiving fluid was collected at different time points (1 h, 2 h, 4 h, 6 h). After the test was completed, the skin was washed and the tissue was then broken up to obtain an intradermal retention extract. The obtained samples were detected by UPLC-MS to obtain the contents of tranexamic acid and tretinoin in the samples.

### 8.2.2 IVPT Results

**Table 12 IVPT Results of Different Compound Preparations**

| **Preparation** | **Tretinoin (ng/cm²)** | | **Tranexamic acid (µg/cm²)** | |
|---|---|---|---|---|
| | **Intradermal retention amount per unit area** | **Permeation amount per unit area** | **Intradermal retention amount per unit area** | **Permeation amount per unit area** |
| Control preparation: Commercially available tretinoin ointment | 31.10±4.47 | ND | NA | NA |
| Preparation 7: Adduct compound gel | 222.29±41.63 | ND | 88.31±17.27 | 357.59±73.60 |
| Preparation 10: Adduct-free process gel | 90.29±12.86 | ND | 37.55±7.20 | 318.23±99.34 |

| | | | | |
|---|---|---|---|---|
| ND- indicates not detected. | | | | |

NA indicates not applicable (the commercially available control preparation is a tretinoin single-component preparation free of tranexamic acid, and therefore is not applicable).

The results are shown in Table 12. The tretinoin adduct/tranexamic acid compound gel (Preparation 7) showed a better transdermal effect than the adduct-free process compound gel (Preparation 10) and the commercially available tretinoin ointment. It is indicated that tretinoin needs to first form an adduct with the ligand diethanolamine before it can exert a permeation-enhancing effect.

### EXAMPLE 9 Another Type of Tretinoin Complex-Tretinoin Triethanolamine Complex

### 9.1 Preparation of Tretinoin-Triethanolamine Complex

**Raw materials and reagents**: Retinoic acid (RA) (AR, Shanghai Aladdin Biochemical Technology Co., Ltd., China), triethanolamine (TEA) (AR, Sinopharm Chemical Reagent Co., Ltd., China), and ethanol (AR, Sinopharm Chemical Reagent Co., Ltd., China).

### Prescription:

| **Material** | **Amount** | **Solvent** | **Solvent amount** |
|---|---|---|---|
| Retinoic acid (RA) | 6.0 g | Ethanol | 30 mL-60 mL |
| triethanolamine (TEA) | 23.87 g | Ethanol | 10 mL |

A molar ratio of triethanolamine (TEA) to retinoic acid (RA) was 8 : 1 (abbreviated as 8[TEA][RA]).

### Process:

(1) Retinoic acid was weighed out according to the prescribed amount and placed in a round-bottom flask, and ethanol was added thereto and the resulting mixture was mixed by stirring. Retinoic acid could not be completely dissolved and was present in a suspended state; and the round-bottom flask was wrapped with aluminum foil to block light.
(2) Triethanolamine was weighed out according to the prescribed amount and placed in a container wrapped with aluminum foil, and ethanol was added thereto, followed by stirring to dilute.

The triethanolamine dilution obtained in step (2) was added dropwise to the mixture obtained in step (1) with stirring; the container was washed with about 5 mL of ethanol, and the resulting liquid was also added to the flask while stirring.

After all the additions were completed, the solution should be present in a clear orange-red color. The stirring was continued for 4 h.

The solvent was removed using a rotary evaporator. The obtained sample was stored in a sealed, light-blocked container.

The ¹H NMR spectrum data of 8[TEA][RA] is as follows:
¹H NMR (400 MHz, DMSO-*d*₆) δ 6.98 (dd, *J* = 15.1, 11.4 Hz, 1H), 6.38 (d, *J* = 15.1 Hz, 1H), 6.26 - 6.15 (m, 2H), 5.77 (s, 1H), 4.45 (s, 24H), 3.41 (td, *J* = 6.1, 0.9 Hz, 48H), 2.55 (td, *J* = 6.1, 0.9 Hz, 48H), 2.26 (d, *J* = 1.0 Hz, 3H), 1.99 (d, *J* = 12.7 Hz, 5H), 1.69 (s, 3H), 1.63 - 1.52 (m, 2H), 1.48 - 1.40 (m, 2H), 1.01 (d, *J* = 0.8 Hz, 6H).

The ¹H NMR spectrum is shown in FIG. 3. As can be seen from the spectrum, the active hydrogen in the carboxyl group of retinoic acid disappeared, and the chemical shift and peak shape of the hydroxyl hydrogen of triethanolamine changed, indicating that retinoic acid and triethanolamine interact to form a complex.

### 9.2 Preparation of Tranexamic Acid/Tretinoin-Diethanolamine Adduct Compound Gel (Preparation 11)

### Prescription:

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.26% |
| Oil phase | Tretinoin-triethanolamine adduct | / | 0.5% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

**Process**: Each adjuvant was weighed out according to the prescribed amount; tranexamic acid was dissolved in about 40% of water. After sodium methylparaben and sodium propylparaben were dissolved in the remaining water, Carbopol 980 was added thereto, and the resulting mixture was stirred at a high rate of 200 rpm for 5 min to disperse uniformly, and stirred at a low rate of 50 rpm for 50 min to hydrate the carbomer. The tranexamic acid solution was added thereto, and mechanically stirred for 30 min to mix uniformly and the carbomer was gelled. The oil phase adjuvants were added thereto, and the resulting mixture was mixed uniformly by stirring to form a homogeneous oil phase. The aqueous phase was added to the oil phase, and the resulting mixture was homogenized with a high-shear homogenizer at 5000 rpm for 3 min. The resulting mixture was mechanically stirred at a low rate for 30 min to form a homogeneous gel. The gel was filled into aluminum-plastic composite tubes.

### 9.3 Detection of Content and Content Uniformity of Tranexamic Acid/Tretinoin-Triethanolamine Adduct compound Gel

Six tubes of a tranexamic acid/tretinoin-triethanolamine adduct compound gel were randomly selected, and the content and content uniformity thereof were measured.

**Table 13 Content and Content Uniformity of Tranexamic Acid/Tretinoin-Triethanolamine Adduct Compound Gel**

| **Name** | **Tretinoin content** | | **Tranexamic acid content** | |
|---|---|---|---|---|
| | **Mean/%** | **RSD%** | **Mean/%** | **RSD%** |
| Preparation 11 | 98.86 | 4.37% | 99.63 | 3.12% |

The results in Table 13 show that the content and content uniformity of the two drugs in the obtained preparation met the standards.

### 9.4 IVPT of Tranexamic Acid/Tretinoin-Triethanolamine Adduct compound Gel

IVPT was conducted on isolated skin from Bama miniature pigs (age ≤ 3 months) in a Franz diffusion cell. The receiving fluid was collected at different time points (1 h, 2 h, 4 h, 6 h). After the test was completed, the skin was washed and then the tissue was broken up to obtain an intradermal retention extract. The obtained samples were detected by UPLC-MS to obtain the contents of tranexamic acid and tretinoin in the samples.

**Table 14 IVPT Results of Tranexamic Acid/Tretinoin-Triethanolamine Adduct Compound Gel**

| **Preparation** | **Tretinoin (ng/cm²)** | | **Tranexamic acid (µg/cm²)** | |
|---|---|---|---|---|
| | **Intradermal retention amount per unit area** | **Permeation amount per unit area** | **Intradermal retention amount per unit area** | **Permeation amount per unit area** |
| Preparation 11: TEA adduct compound gel | 300.72±71.02 | ND | 118.43±35.61 | 331.57±66.48 |
| Preparation 7: DEA adduct compound gel | 410.02±81.01 | ND | 148.44±45.21 | 398.11±52.87 |
| Preparation 3: Ordinary compound gel | 38.99±27.28 | ND | 31.23±11.33 | 300.41±99.55 |

As shown in Table 14, compared with Preparation 3-an ordinary compound gel, Preparation 11-a TEA adduct compound gel also had a permeation-enhancing effect.

### EXAMPLE 10 Another Type of Tretinoin Complex-Tretinoin-Triethylamine Complex 10.1 Preparation of Tretinoin Triethylamine Complex

**Raw materials and reagents**: Retinoic acid (RA) (AR, Shanghai Aladdin Biochemical Technology Co., Ltd., China), and triethylamine (AR, Sinopharm Chemical Reagent Co., Ltd., China).

### Prescription:

| **Material** | **Amount** |
|---|---|
| Retinoic acid (RA) | 6.0 g |
| Triethylamine (Et₃N) | 4.05 g |

The molar ratio of triethylamine to retinoic acid (RA) was 2 : 1 (2[Et₃N][RA]).

**Process:** Retinoic acid was weighed out according to the prescribed amount and placed in a flask wrapped with aluminum foil to block light. A prescribed amount of triethylamine was added thereto. The mixture resulting was sonicated for 30 min until the solution was clear, and further stirred for 4 h to obtain a clear, orange-yellow liquid.

The ¹H NMR spectrum data of 2[Et₃N][RA]:
¹H NMR (400 MHz, DMSO-*d*₆) δ 6.96 (dd, *J* = 15.1, 11.4 Hz, 1H), 6.37 (d, *J* = 15.0 Hz, 1H), 6.29 - 6.18 (m, 2H), 6.15 (d, *J* = 16.1 Hz, 1H), 5.77 (s, 1H), 2.57 - 2.52 (m, 12H), 2.32 - 2.21 (m, 3H), 1.99 (d, *J* = 13.7 Hz, 5H), 1.68 (s, 3H), 1.63 - 1.52 (m, 2H), 1.52 - 1.40 (m, 2H), 1.05 - 0.93 (m, 24H).

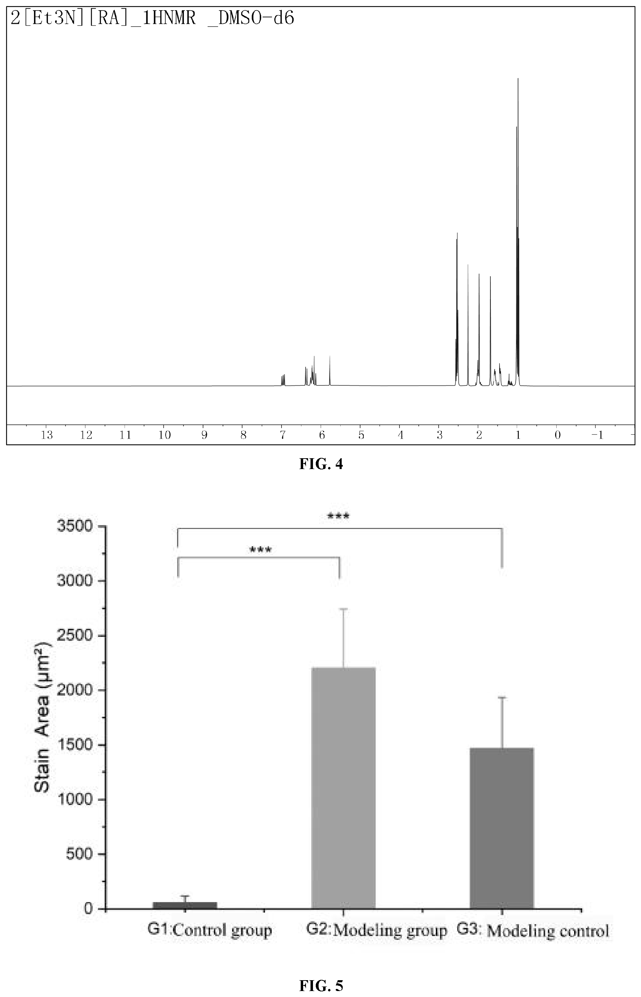

The ¹H NMR spectrum is shown in FIG. 4. In the spectrum, it was observed that the active hydrogen in the carboxyl group of retinoic acid disappeared, and the hydrogen chemical shift in the methylene group of triethylamine moved to a lower field, indicating that retinoic acid and triethylamine interact to form a complex.

### 10.2 Preparation of Tranexamic Acid/Tretinoin-Triethylamine Adduct Compound Gel (Preparation 12)

### Prescription:

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.585% |
| Oil phase | Tretinoin-triethylamine adduct | / | 0.175% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

**Process**: Each adjuvant was weighed out according to the prescribed amount; tranexamic acid was dissolved in about 40% of water. After sodium methylparaben and sodium propylparaben were dissolved in the remaining water, Carbopol 980 was added thereto, and a resulting mixture was stirred at a high rate of 200 rpm for 5 min to disperse uniformly, and stirred at a low rate of 50 rpm for 50 min to hydrate the carbomer. The tranexamic acid solution was added thereto, and the resulting mixture was mechanically stirred for 30 min to mix uniformly, and the carbomer was gelled. The oil phase adjuvants were added thereto and the resulting mixture was mixed uniformly by stirring, thereby forming a homogeneous oil phase. The aqueous phase was added to the oil phase, and the resulting mixture was homogenized with a high-shear homogenizer at 5000 rpm for 3 min. The resulting mixture was mechanically stirred at a low rate for 30 min to form a homogeneous gel. The gel was filled into aluminum-plastic composite tubes.

### 10.3 Measurement of Content and Content Uniformity of Tranexamic Acid/Tretinoin-Triethylamine Adduct compound Gel

Six tubes of a tranexamic acid/tretinoin-triethylamine adduct compound gel were randomly selected, and the content and content uniformity thereof were measured.

**Table 15 Content and Content Uniformity of Tranexamic Acid/Tretinoin-Triethylamine Adduct Compound Gel**

| **Name** | **Tretinoin content** | | **Tranexamic acid content** | |
|---|---|---|---|---|
| | **Mean/%** | **RSD%** | **Mean/%** | **RSD%** |
| Preparation 12 | 99.89 | 3.56% | 99.77 | 3.64% |

The results in Table 15 show that the content and content uniformity of the two drugs in the obtained preparation met the standards.

### 10.4 IVPT of Tranexamic Acid/Tretinoin-Triethylamine Adduct Compound Gel

IVPT was conducted on isolated skin from Bama miniature pigs (age ≤ 3 months) in a Franz diffusion cell. The receiving fluid was collected at different time points (1 h, 2 h, 4 h, 6 h). After the tests were completed, the skin was washed and then the tissue was broken up to obtain an intradermal retention extract. The obtained samples were detected by UPLC-MS to obtain the contents of tranexamic acid and tretinoin in the samples.

**Table 16 IVPT Results of Tranexamic Acid/Tretinoin-Triethylamine Adduct Compound Gel**

| **Preparation** | **Tretinoin (ng/cm²)** | | **Tranexamic acid (µg/cm²)** | |
|---|---|---|---|---|
| | **Intradermal retention amount per unit area** | **Permeation amount per unit area** | **Intradermal retention amount per unit area** | **Permeation amount per unit area** |
| Preparation 12: Triethylamine adduct compound gel | 334.22±70.12 | ND | 123.43±28.61 | 351.37±64.88 |
| Preparation 7: DEA adduct compound gel | 420.12±71.91 | ND | 138.42±47.37 | 387.51±48.33 |
| Preparation 3: Ordinary compound gel | 50.19±28.18 | ND | 65.13±15.02 | 230.28±89.25 |

As shown in Table 16, compared with Preparation 3-an ordinary compound gel, Preparation 12-a triethylamine adduct compound gel also had a permeation-enhancing effect, but the permeation-enhancing effect was slightly junior to that of the DEA adduct compound gel. **EXAMPLE 11 Compound Prescription of Tranexamic Acid/Tretinoin-Diethanolamine Adduct**

In the present disclosure, preparations 13-27 including tranexamic acid and a retinoic acid adduct (a retinoic acid-diethanolamine complex (2[DEA][RA])) were also prepared, with different permeation enhancers, different cosolvents, different antioxidants, and different preservatives, and different drug loading capacities.

### 11.1 Different Permeation Enhancers

### Preparation 13

| **Ingredient** | | | **Proportion** |
|---|---|---|---|
| | Tranexamic acid | Yantai Valiant | 5% |
| | Carbopol 980 | Lubrizol | 1% |
| Aqueous phase | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Glyceryl monocaprylate | IMCD | 2% |
| | Propylene glycol | Shandong Ruisheng | 15% |
| Total | | | 100% |

### Preparation 14

| **Ingredient** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Transcutol P | Gattefossé | 2% |
| | Propylene glycol | Shandong Ruisheng | 15% |
| Total | | | 100% |

### Preparation 15

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Isopropyl myristate | Macklin | 2% |
| | Propylene glycol | Shandong Ruisheng | 15% |
| Total | | | 100% |

### 11.2 Different Cosolvents

### Preparation 16

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | Glycerol | Shandong Ruisheng | 15% |
| Total | | | 100% |

### Preparation 17

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | 1,2-Pentanediol | Shandong Ruisheng | 15% |
| Total | | | 100% |

### 11.3 Different Antioxidants

### Preparation 18

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.59% |
| Oil phase | Tretinoin adduct | / | 0.17% (0.1% in terms of tretinoin) |
| | BHA butylated hydroxyanisole | Jiangxi Alpha | 0.2% |
| | Laurocapram | Shandong Ruisheng | 2% |
| | Propylene glycol | Shandong Ruisheng | 15% |
| Total | | | 100% |

| | | | |
|---|---|---|---|
| **Preparation 19** | | | |

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Sodium pyrosulfite | Sinopharm | 0.2% |
| | Water | / | 76.59% |
| Oil phase | Tretinoin adduct | / | 0.17% (0.1% in terms of tretinoin) |
| | Laurocapram | Shandong Ruisheng | 2% |
| | Propylene glycol | Shandong Ruisheng | 15% |
| Total | | | 100% |

### Preparation 20

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Ascorbic acid | Sinopharm | 0.2% |
| | Water | / | 76.59% |
| Oil phase | Tretinoin adduct | / | 0.17% (0.1% in terms of tretinoin) |
| | Laurocapram | Shandong Ruisheng | 2% |
| | Propylene glycol | Shandong Ruisheng | 15% |
| Total | | | 100% |

### 11.4 Different Preservatives

### Preparation 21

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Water | / | 76.43% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| | Benzoic acid | Sinopharm | 0.2% |
| Total | | | 100% |

### Preparation 22

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Water | / | 76.43% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| | Phenoxyethanol | Nanjing Chemical Reagent | 0.2% |
| Total | | | 100% |

### Preparation 23

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Water | / | 76.59% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| | Methylparaben | Jiangxi Alpha | 0.02% |
| | Propylparaben | Jiangxi Alpha | 0.02% |
| Total | | | 100% |

### 11.5 Different Drug Loading Capacities

### Preparation 24

| **Ingredient/Manufacturer** | | | **Proportion** |
|---|---|---|---|
| Aqueous phase | Tranexamic acid | Yantai Valiant | 7.5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 74.09% |
| Oil phase | Tretinoin adduct | / | 0.17% (0.1% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

### Preparation 25

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.675% |
| Oil phase | Tretinoin adduct | / | 0.085% (0.05% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

### Preparation 26

| **Ingredient/Manufacturer** | | | **Proportion** |
|---|---|---|---|
| Aqueous phase | Tranexamic acid | Yantai Valiant | 2.5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 79.217% |
| Oil phase | Tretinoin adduct | / | 0.043% (0.025% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

### Preparation 27

| **Ingredient/Manufacturer** | | | **Proportion** |
|---|---|---|---|
| Aqueous phase | Tranexamic acid | Yantai Valiant | 5% |
| | Carbopol 980 | Lubrizol | 1% |
| | Sodium methylparaben | Jiangxi Alpha | 0.02% |
| | Sodium propylparaben | Jiangxi Alpha | 0.02% |
| | Water | / | 76.743% |
| Oil phase | Tretinoin adduct | / | 0.017% (0.01% in terms of tretinoin) |
| | Dibutyl hydroxytoluene | Jiangxi Alpha | 0.2% |
| | Laurocapram | Shandong Ruisheng | 2% |
| | Propylene glycol | Shandong Ruisheng | 15% |
| Total | | | 100% |

### EXAMPLE 12: Therapeutic Effect of Example Adduct Compound Preparations on Chloasma

### 12.1. Test System

### 12.1.1. Experimental Animals

### 12.1.1.1. Basic Information of Animals

| | |
|---|---|
| Species/Strain | C57BL/6J mice |
| Grade | SPF |
| Gender and quantity | Female/50 mice |
| Age and weight | 6-8 weeks old/18-22 g |

### 12.1.1.2. Quarantine and Feeding in Acclimatization Period

Quarantine period: at least 3 days; the veterinarian completed the animal quarantine within the quarantine period.

Acclimatization period: at least 2 days; during the acclimatization period, the experimenters conducted a routine inspection once daily.

### 12.1.1.3. Feeding and Environment

| | |
|---|---|
| Temperature and humidity | 20-25 °C; RH 40%-70% |
| Ventilation | SPF-grade ventilation system |
| Lighting | Automatic lighting, alternating between light and dark every 12 hours (lighting could be provided during dark periods if needed for testing), lights off at 7 : 00 PM and on at 7 : 00 AM the next day. |
| Feeding | Cages were used for feeding, with not more than 5 animals per cage. The environmental parameters of the animal housing were recorded during the feeding period. No other species of animals were fed in the same room during the test. |

### 12.1.2. Test Drugs

| **Name** | **Dosage strength** |
|---|---|
| Commercially available progesterone injection | 20 mg/mL |
| Commercially available hydroquinone emulsifiable paste | 2%, 10 g/tube |
| Commercially available tretinoin emulsifiable paste | 0.1%, 10 g/tube |
| Blank adjuvant | 10 g/tube |
| Compound preparation (Preparation 24) | RA 0.1%/TA 7.5%, 10 g/tube |

### 12.2. Test Content

### 12.2.1. Animal Grouping and Treatment Method

| **Group** | **The Number of Animals** | **Drug administered** | **Dose** | **Route of administration** | **Frequency of administration** |
|---|---|---|---|---|---|
| G1: Control group | 11 | NA | NA | NA | NA |
| G2: Model group | 11 | Blank adjuvant | 30 mg/cm² | Topical application | Q.D |
| G3: Modeling control | 5 | Hydroquinone emulsifiable paste | 30 mg/cm² | Topical application | Q.D |
| G4: Positive control | 6 | Hydroquinone emulsifiable paste | 30 mg/cm² | Topical application | Q.D |
| G5: Tretinoin single-component | 6 | Tretinoin cream | 30 mg/cm² | Topical application | Q.D |
| G6: Adduct compound | 6 | RA 0.1%/TA 7.5% | 30 mg/cm² | Topical application | Q.D |

**Grouping**: From 50 animals, 45 were screened out and divided into 6 groups: G1 and G2 each involved 11 animals, G3 involved 5 animals, and G4, G5, and G6 each involved 6 animals.

**Hair Removal**: One day before the test, animals were treated with a hair removal cream to remove hair in an area of about 2*3 cm² from the skin on their backs. Hair removal was then performed two to three times per week, depending on the rate of hair growth.

**Progesterone Injection**: Except for animals in group G1, the rest of the animals were subjected to intramuscular injections of progesterone at a dose of 20 mg/kg daily, alternating between the two hind legs, for 4 weeks.

**UV Irradiation**: Except for animals in group G1, the rest of the animals were anesthetized with isoflurane, and laid flat on surgical pads, and their eyes were covered with light-blocking paper. A UV lamp (UVB light source) with a wavelength of 280-320 nm was placed above the back of the animals for irradiation. The height of the light source away from the back of the animals was 12 cm ± 0.5 cm. The irradiation was performed three times a week, lasting 4 min each time, and lasted 4 weeks. 12.2.2. Determination of Chloasma Model

After the first week of animal modeling, the backs of the animals in group G3 were applied with hydroquinone emulsifiable paste once daily at a dose of 30 mg/cm². The rest of the animals received no treatment. After the end of the third week after modeling, five mice were randomly selected from each of G1 and G2, and group G3 was subjected to euthanization.

Skins from irradiated sites on the back of mice were fixed with 10% formalin, embedded in paraffin, and stained with Fontana-Masson staining. The sections were scanned in their entirety using the software Halo. 10 equal-sized fields of view were then randomly selected from the skin region of each image. Finally, the melanin area of the selected 10 regions was calculated and analyzed using the software.

As shown in FIG. 5, the melanin level of group G2 was significantly higher than that of the control group, and the melanin level of group G3 was also higher than that of the control group but lower than that of the modeling group. It is indicated that progesterone injection and ultraviolet irradiation caused abnormal melanin metabolism and pigmentation in mice, and a chloasma model was successfully established.

### 12.2.3. Efficacy Experiment

Animals in each group were administered with drugs starting at the fourth week according to the grouping design. Three weeks after administration, the test animals were euthanized. Skin from the irradiated area on the back of mice was fixed with 10% formalin, embedded in paraffin, and stained with Fontana-Masson staining. The sections were scanned in their entirety using the software Halo. 10 equal-sized fields of view were then randomly selected from the skin region of each image. Finally, the melanin area of the selected 10 regions was calculated and analyzed using the software.

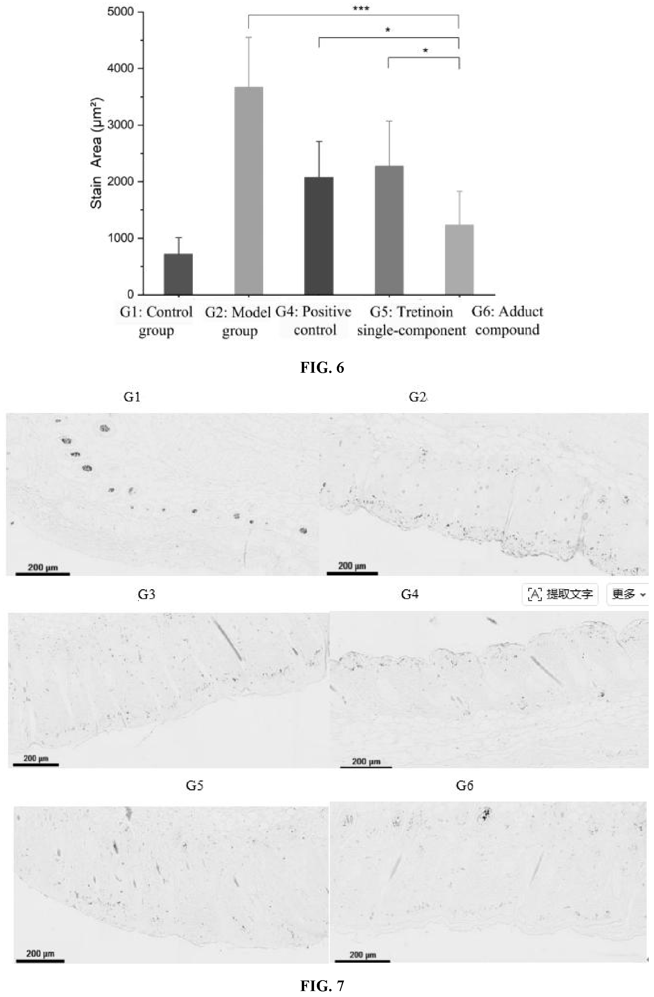

The test results are shown in FIG. 6. Compared with the modeling group, the adduct compound preparation group showed a significant reduction in pigmentation (p < 0.001). Hydroquinone is a first-line drug for the treatment of chloasma. Compared with the hydroquinone group (positive control), the adduct compound preparation showed superior efficacy (p < 0.05); compared with the tretinoin single-component preparation group, the adduct compound preparation showed superior efficacy (p < 0.05). Therefore, the compound preparations of the present disclosure had a good therapeutic effect on chloasma.

### 12.2.4. Typical Section Photographs

Typical section photographs for each group are shown in FIG. 7.

### EXAMPLE 13: Toxicity Study of Adduct Compound Preparations After 4 Weeks of Repeated Administration

### 13.1. Test System

### 13.1.1. Experimental Animals

### 13.1.1.1. Basic Information of Animals

| | |
|---|---|
| Species/Strain | Bama miniature pigs |
| Grade | Ordinary |
| Gender and quantity | Female/20; Male/20 |
| Age and weight | 6-7 months old/11.8-17.4 kg |

### 13.1.1.2. Quarantine and Feeding in Acclimatization Period

Quarantine period: during the housing period, the veterinarian completed the animal quarantine within the quarantine period.

Acclimatization Period: 8 days (including the day of grouping); during the acclimatization period, the experimenters conducted general observations once daily.

### 13.1.1.3. Feeding and Environment

| | |
|---|---|
| Temperature and humidity | 18-26 °C; RH 40%-70% |
| Ventilation | Air exchange times per hour ≥ 8 times, using 100% fresh air (no air circulation). |
| Lighting | Yellow light, automatic lighting, alternating between light and dark every 12 hours (lighting could be provided during dark periods if needed for testing), lights off at 7 : 00 PM and on at 7 : 00 AM the next day. |

### 13.1.2. Test Drug of Each Group

| **Group** | **Drug** | **Dosage strength** |
|---|---|---|
| G1: Negative control group | Physiological saline | 0.9% sodium chloride injection |
| G2: Excipient control group | Blank adjuvant | / |
| G3: Test sample group | Adduct compound preparation (Preparation 7) | RA 0.1%/TA 5.0%, 10 g/tube |
| G4: Commercially available control group | Commercially available tretinoin emulsifiable paste | 0.05%, 20 g/tube |

### 13.2. Test Content

40 Bama miniature pigs were randomly divided into four groups (10 in each group, half male and half female). The drugs were applied to the back skin on both sides of the spine. The animal body surface area to be applied was about 10% of the total body surface area. Specifically, the doses for the negative control group, the excipient control group, the adduct compound group, and the commercially available control group are each 15 mg/cm². The frequency of administration was once a day for 4 consecutive weeks (28 times in total), followed by a 4-week recovery period. Detailed administration design is shown in the table below.

| **Group** | **The number of Animals (Female/Male)** | **Dose** | **Route of administration** | **Frequency of administration** |
|---|---|---|---|---|
| G1: Negative control group | 5/5 | 15 mg/cm² | Topical application | NA |
| G2: Excipient control group | 5/5 | 15 mg/cm² | Topical application | Q.D |
| G3: Test sample group | 5/5 | 15 mg/cm² | Topical application | Q.D |
| G4: Commercially available control group | 5/5 | 15 mg/cm² | Topical application | Q.D |

The toxicity evaluation endpoints of this test included: death/near death, general observation, observation of irritancy response at the administration site, body weight, food intake, ophthalmological examination, body temperature, electrocardiogram (lead II), hematological/coagulation/serum biochemical indexes, and urinalysis. Gross necropsy examination, organ weight and coefficients, and histopathological examination were performed on all animals scheduled for necropsy at the end of the administration period (D29) and the end of the recovery period (D59). Blood samples were collected at different time points before and after the first administration and the last administration for toxicokinetic studies.

### 13.3. Test Results and Conclusions

### 13.3.1 Summary of Irritancy and Pathology

During the test, no significant toxicity changes related to the test sample administered were observed in animal weight, food intake, body temperature, electrocardiogram, ophthalmological examination, hematological/coagulation/serum biochemical indexes, urinalysis, or organ weight.

General observations showed that 4/10 animals of the excipient control group and 4/10 animals of the test sample group exhibited minor to mild skin irritancy on their backs; mild skin irritancy was also observed on the back of 5/10 animals of the commercially available control group. The above findings were recovered at the end of the recovery period.

Gross necropsy revealed that at the end of the administration period, gross necropsy abnormalities related to the test sample or the commercially available control samples were observed at the administration site; minor skin lesions were observed in a few animals of the test sample group, and minor to moderate skin lesions were also observed in a few animals of the commercially available control groups. No gross necropsy abnormalities related to the test samples were observed by the end of the recovery period.

Histopathological examination revealed that histopathological changes associated with the test samples or the commercially available control samples were observed at the administration site. In the test sample group, minor to mild skin lesions were observed. In the commercially available control groups, minor to moderate crusting, moderate erosion/ulceration, and moderate dermal fibrosis were observed.

The above irritancy observation and pathological results at administration sites of the test samples showed that the irritancy by each dosage strength of the test samples was lower than that of the commercially available control sample (0.05% tretinoin cream), and no new changes were observed. It indicates that the above test sample group has a lower risk of local toxic side effects than existing therapeutic drugs.

### EXAMPLE 14: Sensitization Study of Adduct Compound Preparations

### 14.1. Test System

### 14.1.1. Experimental Animals

### 14.1.1.1. Basic Information of Animals

| | |
|---|---|
| Species/Strain | Guinea pig, Hartley-Dunkin |
| Grade | SPF |
| Gender and quantity | Female/25; Male/25 |
| Age and weight | 5-6 weeks old/293-340 g |

### 14.1.1.2. Quarantine and Feeding in Acclimatization Period

Quarantine period: 3 days; the veterinarian completed the animal quarantine within the quarantine period.

Acclimatization Period: 2 days (including the day of grouping); during the acclimatization period, the experimenters conducted cage-side observations once daily and completed one detailed clinical observation before grouping.

### 14.1.1.3. Feeding and Environment

| | |
|---|---|
| Temperature and humidity | 20-26 °C; RH 40%-70% |
| Ventilation | IVC independent air supply system. |
| Lighting | Automatic lighting (using a yellow light lamp), alternating between light and dark every 12 hours (lighting was provided during the dark period for test purposes), lights off at 19 : 00 and on again at 7 : 00 the next day. |

### 14.1.2. Drug Information

| **Name** | **Dosage strength** |
|---|---|
| Physiological saline | 0.9% sodium chloride injection |
| Adduct compound preparation (Preparation 7) | RA 0.1%/TA 5.0%, 10 g/tube |
| Adduct compound preparation (Preparation 26) | RA 0.025%/TA 2.5%, 10 g/tube |
| Commercially available tretinoin emulsifiable paste | 0.05%, 20 g/tube |
| 1-Chloro-2,4-dinitrobenzene | 99.8%, Sigma Aldrich |

### 14.2. Test Content

Fifty guinea pigs were randomly divided into 5 groups. The specific grouping information is shown in the table below.

| **Group** | **The number of Animals (Female/Male)** | **Drug** | **Dosage strength/Concentration (Sensitization)** | **Dosage strength/Concentration (Challenge)** |
|---|---|---|---|---|
| G1: Negative control group | 5/5 | Physiological saline | 0 mg/mL | 0 mg/mL |
| G2: Test sample group | 10/10 | Adduct compound preparation | RA 0.1%/TA 5.0% (Preparation 7) | RA 0.025%/TA 2.5% (Preparation 26) |
| G3: Commercial ly available control group | 5/5 | Commercially available tretinoin emulsifiable paste | 0.05% | 0.05% |
| G4: Positive control group | 5/5 | 1-Chloro-2,4-dini trobenzene | 1% (10 mg/mL) | 0.25% (2.5 mg/mL) |

The route of administration was topical application, with sensitization administration on the 1^{st} day, the 8^{th} day and the 15^{th} day (D1, D8, and D15). Challenge occurred on the 14^{th} day (D29) and the 21^{st} day (D36) after the last sensitization administration.

During the test, it was observed daily whether there were death and near-death cases. Body weight was measured on D-1 (before grouping), D15 (last sensitization), D31 (observation completed 48 h after the first challenge), and D38 (observation completed 48 h after the second challenge), respectively. General observations were performed 1 h and 24 h after sensitization administration (after cleaning the administration site), as well as immediately, 24 h, and 48 h after challenge (after cleaning the administration site) for skin allergic reactions.

### 14.3. Test Results and Conclusions

During the test, no death or near-death cases related to test samples or control samples were observed in any group of animals. Compared with the negative control group, no significant differences in body weight were seen in male and female animals of the low-dose and high-dose test sample groups and the commercially available control group. In the positive control group, male animals showed slow weight gain on D15, D31, and D38, while female animals showed no significant difference in body weight.

In the negative control group, no erythema or edema reactions were observed in the skin after the first challenge administration and the second challenge administration. In the positive control group, erythema or edema reactions were observed in the skin after both the first challenge administration and the second challenge administration, so the incidence of allergic reaction was 100%, indicating that the sensitizing intensity was extremely strong. The test system was deemed valid.

During the sensitization period, the number of the animals in the test sample group that developed erythema 24 h after the second sensitization administration was 1/20, and the number of the animals that developed erythema 1 h and 24 h after the third sensitization administration were 2/20 and 6/20, respectively. Erythema is related to the characteristics of tretinoin in the test sample. During the challenge period, no erythema, edema, or other abnormal reactions were observed in the animals of the test sample group within 48 h after the first challenge and the second challenge. It is indicated that the Buehler test of the test sample on the guinea pigs was negative, that is to say, the adduct compound preparation was non-sensitizing.

During the sensitization period, the number of the animals in the commercially available control group that developed erythema 1 h and 24 h after the second sensitization administration were 5/10 and 3/10, respectively; and the number of the animals that developed erythema 1 h and 24 h after the third sensitization administration were 6/10 and 5/10, respectively. During the challenge period, no erythema or edema reaction was observed after the first challenge administration. The number of the animals that developed erythema immediately, 24 h, and 48 h after the second challenge were 0/10, 2/10, and 1/10, respectively. The incidence of allergic reaction was 20%, and the sensitization intensity was mild.

In summary, under the conditions of this test, the Buehler test was negative in guinea pigs when being administered with the adduct compound preparation transdermally; the commercially available control tretinoin cream (0.05%) caused mild sensitization. Therefore, it can be considered that the adduct compound preparation is non-sensitizing, and the safety risk in causing allergic reactions is lower than that of existing therapeutic drugs.

The embodiments of the present disclosure have been described above. However, the present disclosure is not limited to the embodiments described above. Any modifications, equivalent replacements, or improvements made within the spirit and principles of the present disclosure shall be included within the scope of the present disclosure.

## Claims

1. A composition comprising:
(1) tranexamic acid or a pharmaceutically acceptable salt thereof; and
(2) a tretinoin-alcoholamine complex or a tretinoin-fatty amine complex;
wherein the tretinoin-alcoholamine complex is composed of tretinoin and an alcohol amine compound; and
the tretinoin-fatty amine complex is composed of tretinoin and a fatty amine compound.

2. The composition as claimed in claim 1, wherein the fatty amine compound is selected from a C1-20 alkylamine compound;
preferably, the tretinoin-fatty amine complex is composed of tretinoin and triethylamine;
preferably, in the tretinoin-fatty amine complex, a molar ratio of the fatty amine compound to tretinoin is in a range of (1-100) : 1;
preferably, a mass ratio of component (1) (in terms of tranexamic acid) to component (2) (in terms of tretinoin) is in a range of 0.01-100 : 0.0001-10.

3. The composition as claimed in claim 1, wherein in (2) the tretinoin-alcoholamine complex, a molar ratio of the alcohol amine compound to tretinoin is in a range of (1-100) : 1;
preferably, the alcohol amine compound is selected from formula I, 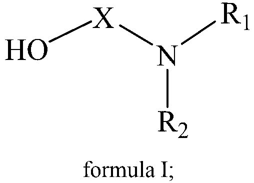
wherein X is selected from C₁₋₆ alkyl (alkylene); 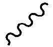
represents a group linkage site; and
R₁ and R₂ are each independently selected from the group consisting of H, C₁₋₆ alkyl, and C₁₋₆ alkyl-OH.

4. The composition as claimed in any one of claims 1-3, wherein in the composition, component (1) and component (2) are each administered in a form of a pharmaceutical composition;
preferably, in the composition, the component (1) and the component (2) are administered simultaneously, sequentially, or at an interval;
preferably, a route of administration of the composition comprises, but is not limited to, gastrointestinal administration or non-gastrointestinal administration;
preferably, the composition comprises, but is not limited to, a pharmaceutical preparation, a cosmetic, a care product, and a beauty product; and
preferably, the composition is administered topically through the skin.

5. The composition as claimed in any one of claims 1-4, wherein in the composition, a content of component (1) is in a range of 0.01%-20% (in terms of tranexamic acid);
preferably, in the composition, a content of component (2) is in a range of 0.001%-2% (in terms of tretinoin);
preferably, the composition is in a dosage strength of 1 g/tube-20 g/tube;
preferably, the composition further comprises:
(3) a gel matrix;
preferably, the gel matrix is selected from carbomer; preferably, a content of the gel matrix is in a range of 0.01%-10%;
preferably, the composition further comprises:
(4) a permeation enhancer;
preferably, the permeation enhancer is at least one selected from the group consisting of glyceryl monocaprylate, Transcutol P, isopropyl myristate, laurocapram, diethylene glycol monoethyl ether, polyglycerol fatty acid ester, propylene glycol monocaprylate, and caprylocaproyl polyoxylglyceride; preferably, a content of the permeation enhancer is in a range of 0.01%-20%;
preferably, the composition further comprises:
(5) a preservative;
preferably, the preservative is at least one selected from the group consisting of benzoic acid, phenoxyethanol, sodium benzoate, methylparaben, ethylparaben, propylparaben, sodium methylparaben, and sodium propylparaben; preferably, a content of the preservative is in a range of 0.001%-2%;
preferably, the composition further comprises:
(6) an antioxidant;
preferably, the antioxidant is at least one selected from the group consisting of butylated hydroxyanisole (BHA), dibutyl hydroxytoluene, butylated hydroxyanisole, vitamin C (ascorbic acid), vitamin E, and sodium pyrosulfite; preferably, a content of the antioxidant is in a range of 0.001%-2%;
preferably, the composition further comprises:
(7) a cosolvent;
preferably, the cosolvent is selected from an alcohol, the alcohol preferably being at least one selected from the group consisting of glycerol, propylene glycol, 1,2-pentanediol, ethanol, and benzyl alcohol; preferably, a content of the cosolvent is in a range of 0.5%-30%;
preferably, the composition further comprises:
(8) an emulsifier;
preferably, the emulsifier is at least one selected from the group consisting of Pemulen TR-1 NF, Emulium Delta MB, Tween, Span, a polyoxyethylene castor oil derivative, poloxamer, Triton, caprylocaproyl polyoxylglyceride, polyethylene glycol stearate, polyoxyethylene-8 beeswax, and lauroyl macrogolglyceride;
preferably, a content of the emulsifier is in a range of 0.1%-30%;
preferably, the composition further comprises:
(9) a greasy matrix;
preferably, the greasy matrix is at least one selected from the group consisting of a long-chain fatty alcohol, a long-chain fatty acid, a fatty acid ester, and grease; preferably, the long-chain fatty alcohol is selected from the group consisting of cetyl alcohol and octadecanol; preferably, the long-chain fatty acid is selected from stearic acid; preferably, the fatty acid ester is at least one selected from the group consisting of isopropyl myristate, a medium-chain triglyceride, glyceryl monooleate, glyceryl monolinoleate, propylene glycol dicaprylate/dicaprate, polyoxyethylene oleate glyceride, myristyl myristate, isopropyl palmitate, isopropyl linoleate, dodecanol benzoate, isostearyl isostearate, fatty acid lactate, decyl oleate, and octyl palmitate; preferably, a content of the greasy matrix is in a range of 0.5%-20%;
preferably, the composition further comprises:
(10) a rheology modifier;
preferably, the rheology modifier is selected from xanthan gum; preferably, a content of the rheology modifier is in a range of 0.01%-0.5%;
preferably, the composition further comprises at least one selected from the group consisting of a metal ion complexing agent, a co-emulsifier, and a texture modifier; preferably, the co-emulsifier is selected from propylene glycol monooctanoate; preferably, a content of the co-emulsifier is in a range of 0.1%-5%; preferably, the texture modifier is selected from cyclopentasiloxane; preferably, a content of the texture modifier is in a range of 1%-5%; preferably, the metal ion complexing agent is at least one selected from the group consisting of ethylenediaminetetraacetic acid (EDTA), disodium ethylenediaminetetraacetate (EDTA-2Na), tetrasodium ethylenediaminetetraacetate (EDTA-4Na), disodium nitrilotriacetate, sodium tripolyphosphate, sodium hexametaphosphate, and tetrapotassium pyrophosphate;
preferably, the composition further comprises other physiologically acceptable carriers.

6. The composition as claimed in any one of claims 1-5, wherein the composition comprises the following components:
2.5%-7.5% of tranexamic acid;
0.01%-1% of the tretinoin-alcoholamine complex or the tretinoin-fatty amine complex, in terms of tretinoin;
0.05%-5% of the gel matrix;
optionally, 0.1%-15% of the permeation enhancer;
optionally, 0.005%-1% of the preservative;
optionally, 0.01%-1% of the antioxidant;
optionally, 1%-20% of the cosolvent;
optionally, 0.2%-20% of the emulsifier;
optionally, 1.0%-15% of the greasy matrix;
optionally, 0.1%-0.3% of the rheology modifier;
optionally, 1%-3% of the co-emulsifier;
optionally, 1%-5% of the texture modifier; and
a balance being water.

7. The composition as claimed in any one of claims 1-6, wherein the composition comprises the following components:
| | |
|---|---|
| the tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD 2020 : | 1% or 0.2% or 0.15%; |
| an antioxidant: | 0.1% or 0.2%; |
| a preservative: | 0.02% or 0.04%; |
| a permeation enhancer: | 2%; |
| an emulsifier: | 10%; |
| a cosolvent: | 10%; |
| a balance being water; | |
wherein the tretinoin-alcoholamine complex or the tretinoin-fatty amine complex for example, tretinoin diethanolamine, tretinoin triethanolamine, and tretinoin triethylamine;
preferably, the composition comprises the following components:
| | |
|---|---|
| the tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD2020: | 1% or 0.2% or 0.15%; |
| an emulsifier: | 0.3% or 0.6%; |
| a greasy matrix: | 5%; |
| an antioxidant: | 0.1% or 0.2%; |
| a preservative: | 1%; |
| a permeation enhancer: | 12.57%; |
| a cosolvent: | 10% or 12.5%; |
| a balance being water; | |
wherein the tretinoin-alcoholamine complex or tretinoin-fatty amine complex, for example, tretinoin diethanolamine, tretinoin triethanolamine, and tretinoin triethylamine;
preferably, the composition comprises the following components:
| | |
|---|---|
| the tranexamic acid: | 2.5% or 5% or 7.5%; |
| the tretinoin-alcoholamine complex or the tretinoin-fatty amine complex: | 0.01% or 0.025% or |
| | 0.05% or 0.1%, in terms of tretinoin; |
| Carbopol 980 or Carbopol ETD2020: | 1% or 0.2% or 0.15%; |
| a preservative: | 0.02% or 0.04%; |
| an antioxidant: | 0.1% or 0.2%; |
| a permeation enhancer: | 2%; |
| a cosolvent: | 15%; |
| a balance being water; | |
wherein the tretinoin-alcoholamine complex or the tretinoin-fatty amine complex, for example, tretinoin diethanolamine, tretinoin triethanolamine, and tretinoin triethylamine;
preferably, the composition comprises the following components:
| | |
|---|---|
| the tranexamic acid: | 2.5% or 5% or 7.5%; |
| the tretinoin-alcoholamine complex or the tretinoin-fatty amine complex: | 0.01% or 0.025% or 0.05% or 0.1%, in terms of tretinoin; |
| Carbopol 980 or Carbopol ETD2020: | 1% or 0.2% or 0.15%; |
| a permeation enhancer: | 5.0%; |
| an emulsifier: | 2.0%; |
| a greasy matrix: | 5% or 1.5% or 6.5%; |
| a co-emulsifier: | 2.0%; |
| a texture modifier: | 3.0%; |
| a preservative: | 0.5%; |
| a cosolvent: | 2%; |
| a rheology modifier: | 0.15%; |
| a balance being water; | |
wherein the tretinoin-alcoholamine complex or the tretinoin-fatty amine complex, for example, tretinoin diethanolamine, tretinoin triethanolamine, and tretinoin triethylamine.

8. Use of the composition as claimed in any one of claims 1 to 7 in preparation of a drug for preventing and/or treating a skin disease;
preferably, the skin disease is selected from the group consisting of chloasma and pigmentation.

9. A method of preventing and/or treating a skin disease, comprising:
administering an effective amount of the composition as claimed in any one of claims 1-7 to a patient suffering from a skin disease;
preferably, the skin disease is selected from the group consisting of chloasma and pigmentation.

10. A method of preparing the composition as claimed in any one of claims 1-7, comprising steps of:
1) preparing an aqueous phase containing the tranexamic acid;
2) preparing an oil phase containing the tretinoin-alcoholamine complex or the tretinoin-fatty amine complex; and
3) mixing by adding the aqueous phase into the oil phase, and subjecting a resulting mixture to emulsification and homogenization to obtain the composition.
